# EUROPEAN PATENT APPLICATION

(11) **EP 4 447 643 A1**
(43) Date of publication of application: **16.10.2024**
(21) Application number: 23168712.0
(22) Date of filing: 19.04.2023
(51) Int. Cl.: H10K 85/00, C07D 209/88, C07C 217/92

(54) **IN-SITU CROSSLINKING OF 9,9`-SPIROBIFLUORENE-BASED COMPOUNDS FOR USE IN OPTOELECTRONIC AND/OR PHOTOELECTROCHEMICAL DEVICES AND MANUFACTURE THEREOF**

(30) Priority: 14.04.2023 US 202318134751
(71) Applicant: Kaunas University of Technology, 44029 Kaunas (LT)
(72) Inventor: Getautis, Vytautas, 51390 Kaunas (LT); Daskeviciene, Maryte, 55232 Jonava (LT); Daskeviciute-Geguziene, Sarune, 55461 Jonavos raj., Prauliai (LT); Rakstys, Kasparas, 52193 Kaunas (LT); Wakamiya, Atsushi, Uji, Kyoto, 611-0011 (JP); Truong, Minh Anh, Uji, Kyoto, 611-0011 (JP)
(74) Representative: Klimaitiene, Otilija

(57) **Abstract**

The 9,9'-spirobifluorene-based compounds and mixtures including 9,9'-spirobifluorene compounds with crosslinkable functional groups and compounds with two or more thiol groups, can stabilize one or more underlying layers of the hole transporting layer and/or interlayer during liquid fabrication process of optoelectronic and/or photoelectrochemical devices. More particularly, the compounds are hole transport materials that include crosslinkable functional groups covalently bonded to 9,9'-spirobifluorene hole transporting structure and mixtures including hole transporting crosslinkable 9,9'-spirobifluorene and thiol derivatives, which may crosslink, such as by exposure to UV, visible light, and/or heat. Photovoltaic devices may employ these compounds and mixtures in crosslinked forms.

## Description

### TECHNICAL FIELD

The invention generally relates to 9,9'-spirobisfluorene-based compounds, and their use as hole transport materials in optoelectronic and/or photoelectrochemical devices, and a method of fabrication thereof.

### BACKGROUND OF THE INVENTION

Over the past decades, there has been a strong interest in renewable energy sources, especially the most potent among them-the sun. The conversion of solar energy to electrical current using thin film third-generation photovoltaics (PV) has been widely explored for the last two decades. The sandwich/monolithic-type PV devices, consisting of a mesoporous photoanode with an organic/inorganic light harvester, redox electrolyte/solid-state hole conductor, and counter electrode, have gained significant interest due to the ease of their fabrication, the flexibility in the selection of materials and the low cost of production.

Organic-inorganic metal halide perovskite solar cells (PSCs) have experienced rapid development in recent years, with its power conversion efficiency (PCE) improving from 3.8% in 2009 to an impressive 25.7% in 2021;^{[1]} such values are now comparable with those of market-established solar cell technologies such as crystalline silicon (c-Si) and copper indium gallium diselenide (CIGS).^{[2,3]} Moreover, PSCs can be manufactured using low-cost, scalable, simple solution-processing techniques, further underlining the promise of PSCs as a future mainstream technology.^{[4]} Moreover, PSCs can also be integrated as top cell into tandems when combining either with c-Si, CIGS, organic or another PSC as bottom cell technology, yielding PCEs well above those of the sub-cells, opening pathways toward affordable ultrahigh efficient PV.^{[5]} Despite the fact that perovskite solar cells have demonstrated impressive efficiency there are still considerable obstacles to overcome before the technology can be commercialized. The most important of these is the insufficient stability of the devices, mainly due to decomposition of the perovskite absorber when it is exposed to the ambient environment.

A common PSC device consists of two electrodes, the perovskite light absorber, an n-type electron transport layer (ETL), and a p-type hole transport layer (HTL).^{[6]} The perovskite layer is sandwiched between ETL and HTL for efficient charge transport. The outer electrodes, including transparent conducting glass covered with indium tin oxide (ITO) or fluorine-doped tin oxide (FTO) and counter electrode (Au, Ag or carbon), are used for charge collection. Three types of device configurations are commonly used to fabricate PSCs, including the mesoporous n-i-p, planar n-i-p, and planar p-i-n structure, in which n represents ETL, p represents HTL, and i represents the perovskite layer, respectively.

The n-i-p structure has systematically outperformed its p-i-n counterpart, up to present.^{[7]} Nevertheless, p-i-n PSCs offer several advantages. First, high-temperature sintering (>450 °C, required for the mesoporous TiO₂ formation) is avoided, making this technology of particular appeal for applications with limited temperature resilience, such as most tandem solar cells and flexible devices.^{[8]} Second, no extrinsic dopant is needed for the charge transporting layer (CTL), which can be instrumental towards improved device stability. For instance, it is well known that the dopants in the most commonly used hole transporting material (HTM) Spiro-OMeTAD (2,2',7,7'-tetrakis[N,N-di(4-methoxyphenyl)amino]-9,9'-spirobifluorene) in n-i-p PSCs can accelerate device degradation because of doping-related Li⁺ migration across the perovskite layer; moreover the hygroscopic nature of Spiro-OMeTAD results in facile and undesired moisture ingress into the perovskite.^{[9]} Third, p-i-n PSCs usually feature a much less pronounced hysteresis in their current-voltage characteristics, compared to n-i-p devices.^{[10]} Fourth, the fabrication costs of single-junction p-i-n PSCs can arguably be lower because for the back electrode, a cheaper metal such as Ag, Al, or Cu can be used, compared to Au in the n-i-p structure.

Hole transporting materials is one of the quintessential components required for efficient PV devices. These materials are responsible for the transport of photogenerated carriers from the absorber towards the electrode. HTMs should demonstrate sufficient charge transport properties, suitable energy levels, such that its highest occupied molecular orbital (HOMO) level is higher than -5.7 eV, and good thermal stability. These materials are a weak spot in the whole PV device.

Inorganic HTM (such as NiOx) possess a high thermal stability and transparency, their PV performance is usually inferior to their organic counterparts due to relatively low intrinsic conductivity and surface defects.^{[11]} However, most inorganic HTLs require either a high-temperature or high vacuum processing step and may feature a poor wettability with respect to the perovskite precursor ink, in the case of solution processing. Finally, the available materials suitable as inorganic HTM are limited.

On the contrary, organic HTMs can be solution-processed at low temperature, and their properties can be finely adjusted through molecular design. Moreover, combining novel building blocks through organic synthesis can greatly expand the library of organic HTLs, thus showing potential for further PSC development in terms of performance, stability, and scalability.

Despite significant research efforts devoted towards development of new hole transporting materials, the field is still dominated by 9,9'-spirobifluorene compound spiro-OMeTAD, an organic HTM.^{[12]} The "spiro concept" comprises two extended p-systems that are connected to a tetrahedral sp³-hybridized atom, resulting in an orthogonal configuration between the aromatic rings. This spiro linkage improves the morphological stability, whereas the electronic properties of the electroactive moieties remain intact. Owing to its attractive properties, 9,9'-spirobifluorene has been exploited for organic electronics, mainly for photovoltaic applications.

Spiro-OMeTAD combines the following factors: (1) it has a large band gap (about 3.0 eV) and a relatively deep-lying HOMO energy level, which provides good electronic alignment with perovskite layers, and its band gap can be further tuned to a chosen perovskite's electronic structure. (2) Spiro-OMeTAD has a well-studied synthesis and solution process, which is advantageous for large area fabrication of rigid and flexible solar cells. (3) The melting temperature of spiro-OMeTAD is high, which adds to the thermal stability of the device. (4) The pristine spiro-OMeTAD HTL shows low conductivity and hole mobility. A common method is to use additives such as LiTFSI and 4-tert-butylpyridine (TBP) to enhance the electrical properties of spiro-OMeTAD films. For the above reasons, spiro-OMeTAD based HTLs undoubtedly play an important role in the development of PSCs, especially of n-i-p architecture.^{[12]}

There is significantly less data on the use of spiro-OMeTAD in p-i-n architecture PSCs. Hong Meng, Hongzheng Chen and Wei Huang with coworkers have synthesized three new spiro-bifluorenes as HTMs by replacing the para-methoxy substituent in Spiro-MeOTAD with methylsulfanyl, N,N-dimethylamino and ethyl groups.^{[13]} Surprisingly, the methylsulfanyl substituted spiro-bifluorene shows the highest power conversion efficiency of 15.92% among the investigated spiro-bifluorenes, which is an over 38% increase in PCE compared with that of spiro-MeOTAD under similar device fabrication.

In the case of p-i-n devices, solution-processing of the perovskite absorber layer adds additional constraints on the choice of HTMs, as it usually should withstand a mixture of polar DMF:DMSO solvents. Therefore, so far the most popular choice of organic HTMs for such devices are polymers, such as PEDOT:PSS and PTAA or combination thereof.^{[14,15]} PTAA (poly[bis(4-phenyl)(2,4,6-trimethylphenyl)amine]), which is a polymeric structure of triphenylamines, deserves a special mention as it currently holds the PCE world record for PSCs.^{[16]} However, polymeric structures have certain drawbacks such as batch-to-batch reproducibility, difficult purification, and broad molar mass distribution. In this regard, small organic molecules offer potential advantages such as well-defined molecular weight, easy purification and good reproducibility.

Herein, we disclose the development of novel HTMs, which comprise cross-linkable functional groups covalently bonded to 9,9'-spirobifluorene hole transporting structure, and mixtures including hole transporting, cross-linkable 9,9'-spirobifluorene and thiol derivatives, which may also cross-link by exposure to light and/or heat. These HTMs not only efficiently extract and transport charges, but also can yield 3D insoluble polymers. It was further shown that polymerization can take place at low temperatures, therefore the materials are suitable for the application in both n-i-p and pi-n PSCs architecture. Efficient and stable photovoltaic devices employing these compounds and mixtures in crosslinked forms as hole transporting layers are also disclosed. Additionally, such organic semiconductors are attractive not only for photovoltaics but also for the development of other low-cost, high-performance optoelectronic devices such as light emitting diodes, phototransistors, photocells, and so on.

### SUMMARY OF THE INVENTION

In one embodiment, the present description discloses a 9,9'-spirobifluorene polymer precursor, possessing crosslinkable functional groups and tunable energetic levels, which forms crosslinked polymers by exposure to light and/or heat during liquid fabrication processes. In another embodiment, the present description discloses mixtures comprising 9,9'-spirobifluorene polymer precursors possessing crosslinkable functional groups and a crosslinking agent with two or more thiol groups, such mixtures forming crosslinked polymers by exposure to light and/or heat during liquid fabrication process. The disclosed crosslinked polymers are 3D insoluble polymers which can be used as hole transporting materials. The crosslinked polymers can be used in hole transporting layers and provide benefits ranging from resistance to various environmental effects, including strong solvents, further preventing intermixing of the different active layers, and improves power conversion and stability in devices, in particular in solid state solar cell comprising perovskites. Polymerization using the crosslinking agent takes place at low temperatures, below 110°C, therefore the mixture including the hole transporting compound and thiol derivative is suitable for both n-i-p and p-i-n PSCs architectures and provides increased stability and performance.

The present teachings also

In an aspect, the teachings provide a crosslinkable 9,9'-spirobisfluorene compound that can be used as a new hole transporting materials, which provide higher power conversion efficiency (PCE) to stable photovoltaic devices comprising perovskite, organic, or organometallic dyes as sensitizer.

In another aspect, the invention provides an optoelectronic and/or photoelectrochemical device comprising a hole transporting material comprising the compound of formula (I) or a mixture thereof with a crosslinking agent with two or more thiol groups used as a polymerized film in the device.

In a further aspect, a method for fabricating an optoelectronic and/or photoelectrochemical device comprising a hole transport layer is provided, said method comprising;
- providing a first layer before, wherein the first layer is an underlying layer;
- providing a second layer, wherein the second layer is the hole transport layer, onto the underlying layer, said providing comprising steps of:
- applying a hole transporting material comprising the compound of formula (I) or mixture thereof with a crosslinking agent containing two or more thiol groups, by liquid deposition onto the underlying layer, and
- crosslinking the hole transporting material by thermal, chemical or irradiative means, and
- providing a third layer, wherein the third layer is an overlying layer.

In this aspect, the underlying layer is selected from a hole injection layer, a sensitizer layer, a light-harvester layer, or a conducting current collector; wherein the overlaying layer is selected from an emissive layer when the underlying layer as a hole injection layer, from a counter electrode or a conducting current providing layer when the underlying layer is a sensitizer layer or a light-harvesting layer, or from a sensitizer layer when the underlying layer is a conducting current collector.

Further aspects and preferred embodiments are detailed herein below and in the appended claims. Further features and advantages will become apparent to the skilled person from the description of the preferred embodiments given below.

### BRIEF DESCRIPTION OF THE DRAWINGS

**Fig. 1 a)** shows a schematic diagram of mesoporous n-i-p PSC device structure; **b)** planar n-i-p PSC device structure; c) p-i-n PSC device structure.
**Fig. 2 a)** shows differential scanning calorimetry (DSC) first and second heating curves of HTM1 (scan rate 10 °C/min, N₂ atmosphere); **b)** shows DSC first and second heating curves of HTM1+4,4'-thiobisbenzenethiol, 1:2 (scan rate 10 °C/min, N₂ atmosphere).
**Fig. 3 a)** shows DSC first and second heating curves of HTM2 (scan rate 10 °C/min, N₂ atmosphere); **b)** shows DSC first and second heating curves of HTM2+4,4'-thiobisbenzenethiol, 1:2 (scan rate 10 °C/min, N₂ atmosphere).
**Fig. 4 a)** shows UV/vis spectrum of the solutions prepared by dipping spin coated HTM1 films into THF after heating at cross-linkable temperature for the respective duration; **b)** shows UV/vis spectrum of the solutions prepared by dipping spin coated HTM1+4,4'-thiobisbenzenethiol (1:2) films into THF after heating at cross-linkable temperature for the respective duration.
**Fig. 5 a)** shows UV/vis spectrum of the solutions prepared by dipping spin coated HTM2 films into THF after heating at cross-linkable temperature for the respective duration; b) shows UV/vis spectrum of the solutions prepared by dipping spin coated HTM2+4,4'-thiobisbenzenethiol (1:2) films into THF after heating at cross-linkable temperature for the respective duration.
**Fig. 6 a)** shows photoemission in air spectra of the charge transporting layers with HTM1; **b)** shows photoemission in air spectra of the charge transporting layers with HTM2; c) shows photoemission in air spectra of the charge transporting layers with HTM3.
**Fig. 7 a)** shows electric field dependencies of the hole-drift mobility of the charge transporting layers with HTM1; **b)** shows electric field dependencies of the hole-drift mobility of the charge transporting layers with HTM2; c) shows electric field dependencies of the hole-drift mobility of the charge transporting layers with HTM3.
**Fig. 8** shows a schematic diagram of the investigated p-i-n PSC device structure.
**Fig. 9** shows J-V characteristics of the HTM1 in p-i-n devices.
**Fig. 10** shows IPCE spectra of p-i-n devices containing HTM1.
**Fig. 11** shows a schematic diagram of the investigated n-i-p PSC device structure.
**Fig. 12** shows *J-V* curves and IPCE spectrum of n-i-p devices containing HTM1.
**Fig. 13** shows MPPT tracking of n-i-p devices with and without HTM1 interlayer.

### DETAILED DESCRIPTION OF THE INVENTION

The main object of the present teachings is new compounds of formula **(I)** containing a 9,9'-spirobifluorene center: 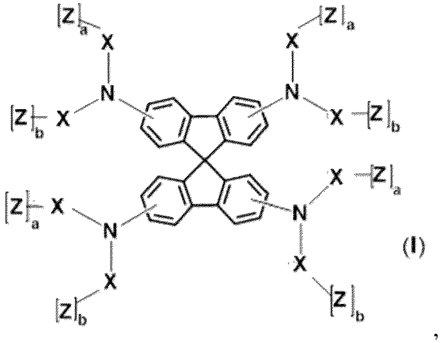 wherein
- X is independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said alkyl, alkenyl, alkynyl moieties, if they comprise 3 or more carbons, may be linear, branched or cyclic, and said alkyl, alkenyl, alkynyl, aryl, alkylaryl, alkenylaryl, alkynylaryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl, C4 to C10 heteroaryl, or one or more heteroatoms being selected from N, S and O;
- *a* and *b* are an integer independently being 0 or 1, wherein *a*+*b* ≥ 1;
- Z is selected from C2-C10 alkenyl, C2-C10 alkynyl, C4-C20 alkenylaryl, acetylenyl group, alkenyloxy alkyl group, -SH, acrylate group, -OH, -COOH, urethane group, ethyl ester group, C4-C20 alkoxyalkenyl, azide group, epoxy compounds, methyl oxirane group, epoxy group, oxiranyl group, and oxetanyl group, wherein Z is independently selected for groups [Z]ₐ and [Z]_{b}.

X may be independently selected from C1-C10 alkyl, C2-C10 alkenyl, C2-C10 alkynyl, C4-C10 aryl, wherein if said alkyl, alkenyl, alkynyl moieties comprise 3 or more carbons, may be linear, branched or cyclic, and said alkyl, alkenyl, alkynyl, aryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl, C4 to C10 heteroaryl, or one or more heteroatoms being selected from N, S and Preferably X may be independently selected from C1-C6 alkyl, C2-C6 alkenyl, C2-C6 alkynyl, C4-C6 aryl, wherein if said alkyl, alkenyl, alkynyl moieties comprise 3 or more carbons, may be linear, branched or cyclic, said moieties being unsubstituted or substituted as defined herein.

In another embodiment, *a* and *b* of the compound of formula **(1)** are equal *a*+*b* ≥ 1. Thus, Z is the part of the crosslinkable part of the compound of formula **(I)** and can be crosslinked with one or more additional compounds of formula **(I).**

Z of the compound of formula **(I)** is independently selected from C2-C10 alkenyl, C2-C10 alkynyl, C4-C20 alkenylaryl, acetylenyl group, alkenyloxy alkyl group, -SH, acrylate group, -OH, -COOH, , urethane group, ethyl ester group, C4-C20 alkoxyalkenyl, azide group, epoxy compounds, methyl oxirane group, epoxy group, oxiranyl group, and oxetanyl group.

In one embodiment, *a* or *b* of the compound of formula **(I)** is 0. Therefore only the single moiety Z of the same branch is present in the compound of formula **(I).** 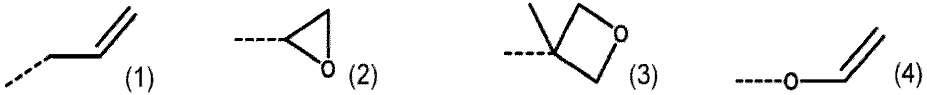 wherein the dotted line represents a single bond between the substituent X of the compound of formula **(I),** when *a* and/or *b* is 1, and Z is anyone of the moieties (1)-(4).

Provided herein is a polymer of compounds of formula **(I)** as defined above, wherein the crosslinkable part or Z moiety is crosslinked to another Z moiety of one or more compounds of formula **(I).** The compounds of formula **(I)** in the polymer are identical or a mixture of different compounds of formula **(I)** with the proviso that the Z moiety is identical between the different compounds of formula **(I).** A compound of formula **(I)** represents one unit or a monomer being polymerizable by photo, thermal or chemical crosslinking to form a polymer during fabrication of the device. Said polymer is insoluble to the overlying layers and protects from the solubilisation of the underlying layer.

Said polymer comprises a compound of general formula **(Ia)** 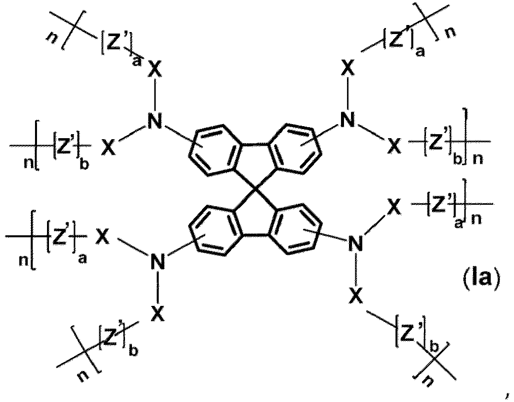 wherein *a, b* and X are defined similarly to formula (I); n is an integer equal to or greater than 2 or 3; and Z' is the Z moiety in crosslinked form with another Z moiety, wherein the crosslinkable moiety, Z is independently selected for groups [Z]ₐ and [Z]_{b} and all Z moieties bonding to another are identical. Preferably n is ≥ 3.

In particular, the moiety 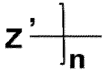 is selected from a moiety according to anyone of formulae (1')-(4') below

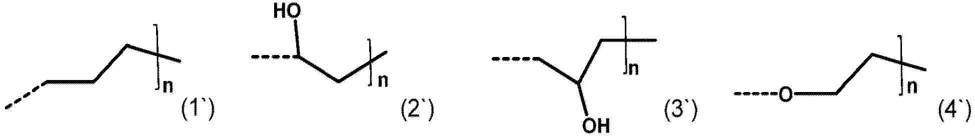

In one example, n is an integer equal to 2 or 3. In another example, n is ≥ 3.

Describe herein is a polymer of the compound of formula **(I)** as defined above, wherein the crosslinkable moiety, Z, is crosslinked to a crosslinking agent comprising two or more thiol groups.

In further preferred embodiments the crosslinking agent with two or more thiol groups is selected from any one of formula (5) to (15) but is not limited to:

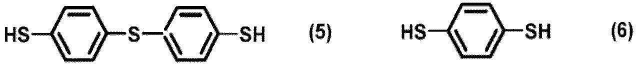

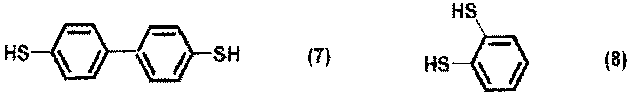

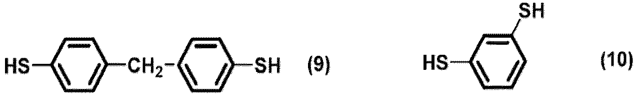

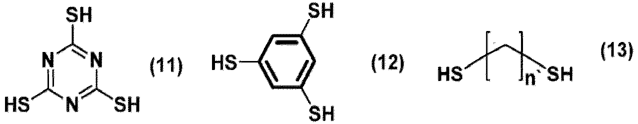

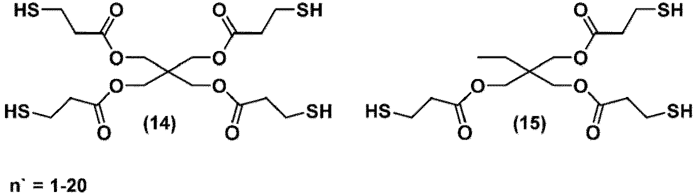

Said polymer comprises a compound of general formula **(Ib)**

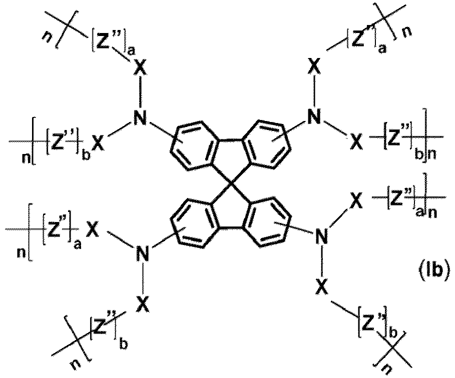

Wherein *a, b* and X are defined herein and as above; n is an integer equal or above 2 or 3; and Z" is selected from the Z moiety, crosslinked with a crosslinking agent with two or more thiol groups. The compound (Ib), being further crosslinked to a Z" moiety of one or more compounds of formula **(I),** having the same moiety Z. Preferably n is ≥ 3.

In particular, the moiety 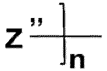 is selected from a moiety according to anyone of formulae (1")-(4") below, when the crosslinking agent is compound (5) e.g.

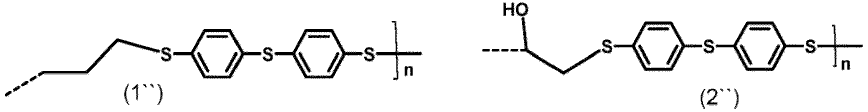

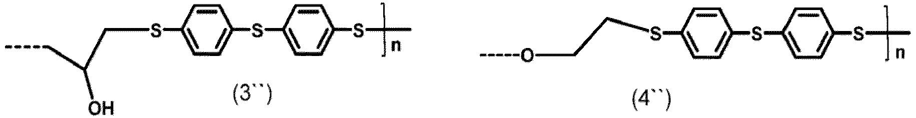

According to another embodiment, the compounds of formula **(I)** containing 9,9'-spirobifluorene center are hole transporting materials selected from, but not limited to, a compound according to any one of formulae **(HTM1)** to **(HTM4):**

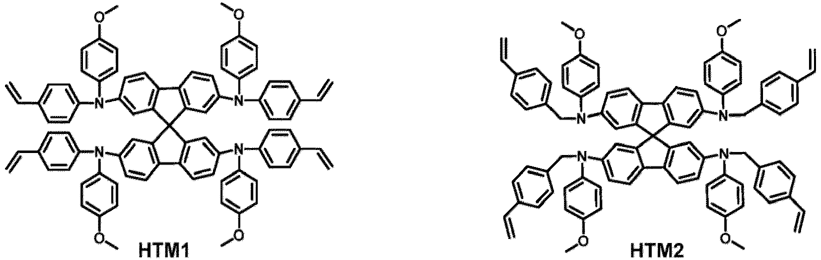

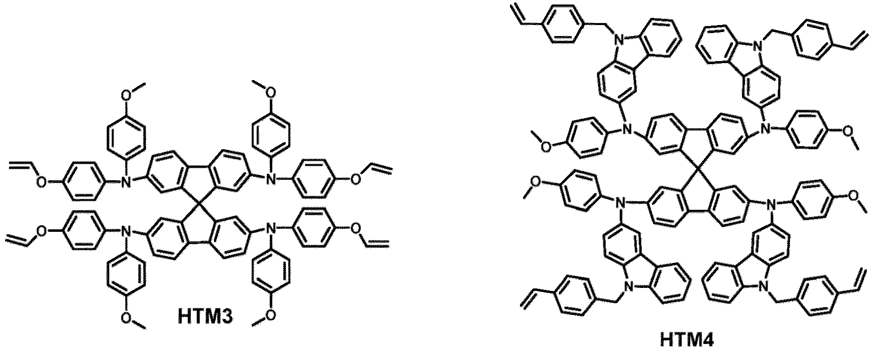

### Optoelectronic and/or photoelectrochemical device

Another embodiment described herein is an optoelectronic and/or photoelectrochemical device comprising a compound of formula **(I)** in a polymerized film. The crosslinkable moiety, Z, of the compound of formula **(I),** is crosslinked to another Z moiety of one or more additional compounds of formula **(I)** to form the polymer film. The monomers or units in the polymer are linked to each other by modified Z moieties such as illustrated by the specific moieties, Z', of formulae (1')-(4') or Z" moieties of formulae (1"- 4").

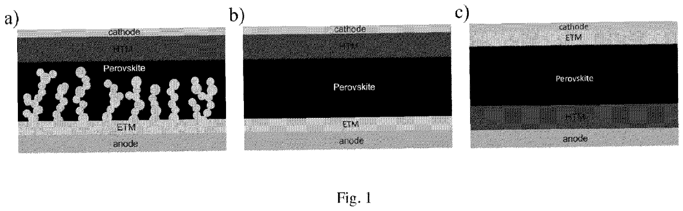

An optoelectronic and/or photoelectrochemical device is a photovoltaic device, a solid state solar cell or a perovskite solar cell. Said perovskite solar cell is selected from a n-i-p perovskite solar cell or a p-i-n perovskite solar cell. In particular, as illustrated in Fig. 1 a and b, the n-i-p perovskite solar cell may comprise a conducting current collector or conductive substrate (anode), n-type semiconductor (ETM) or an optional n-type surface-increasing mesoporous structure, a sensitizer or light-harvesting layer (Perovskite), a hole transport layer (HTM), and conductive counter electrode (cathode). As illustrated in Fig. 1 c, the p-i-n perovskite solar cell may comprise a conducting substrate layer (anode), a hole transport layer (HTM), a sensitizer or light-harvesting layer (Perovskite), n-type semiconductor (ETM), and metallic counter electrode (cathode). Said devices comprise two opposite sides: one side exposed to the light and one side without direct illumination, towards which either electrons or holes are directed according to the energy levels between the different layers.

The conducting current collector or substrate layer is preferably substantially transparent. "Substantially transparent" means transparent to at least a part, preferably a major part of the visible light. Preferably, the conducting support layer is substantially transparent to all wavelengths or types of visible light. Furthermore, the conducting support layer may be transparent to non-visible light, such as UV and IR radiation, for example. The current collector is a conducting support layer providing the support layer of the solar cell. Preferably, the solar cell is built on said support layer.

According to an embodiment, the solar cell preferably comprises one or more support layers. The support layer preferably provides the physical support of the device. Furthermore, the support layer preferably provides protection with respect to physical damage and thus delimits the solar cell with respect to the outside, for example on at least one of two major sides of the solar cell. The support layer is not specifically shown in the figures. In some embodiments, a glass or plastic coated transparent conductive oxides (TCO) current collector is used. These materials, which are commercially available, contain the support as well as the current collector.

According to another embodiment, the support of the solar cell may be provided on the side of the counter electrode or the hole selective back contact. In this case, the conductive support layer does not necessarily provide the support of the device but may simply be or comprise a current collector, for example a metal foil.

The conducting current collector or substrate, collecting the current obtained from the solar cell may comprise a conducting or semiconducting material, such as a conducting organic or inorganic material, such as a metal, doped metal, a conducting metal oxide or doped metal oxide, for example.

The conducting current collector or substrate layer comprises a material selected from indium doped tin oxide (ITO), fluoride doped tin oxide (FTO), ZnO-Ga₂O₃, ZnO-Al₂O₃, tin-oxide, antimony doped tin oxide (ATO), SrGeO₃ and zinc oxide or combinations thereof, which may be coated on a transparent substrate, such as plastic or glass. In this case, the plastic or glass provides the support structure of the layer and the cited conducting material provides the conductivity. Such support layer are generally known as conductive glass and conductive plastic, respectively, which are thus preferred conducting support layers.

According to another embodiment, the conducting support layer comprises a conducting transparent layer, which may be selected from conducting glass and conducting plastic. The conducting current collector is preferably arranged to collect and conduct the current generated in the working electrode or photoanode. In another embodiment, the current collector is in electric contact with the n-type semiconductor layer or conductive anode, in particular in the case of a n-i-p solar cell.

The n-type semiconductor layer or conductive anode comprises n-type semiconductor materials selected from n-type metal oxides or n-type semiconducting polymers. The n-type metal oxides may be selected from Ti, Sn, Fe, Zn, W, Mo, Nb, SrTi, Si, Ti, Al, Cr, Sn, Mg, Mn, Zr, Ni and Cu. The n-type semiconducting polymers may be selected from PCBM-like C60 and fullerene derivatives selected from [6,6]-phenyl-C₆₁-butyric acid methyl ester PCBM-like C60 or PCBM), 1,4,5,8,9,11-hexazatriphenylene-hexacarbonitrile (HAT-CN), (C₆₀-Iₕ)[5,6]fullerene (C60), (C70-D5h)[5,6]fullerene (C70), [6,6]-Phenyl C₇₁ butyric acid methyl ester (PC70BM), 2,9-dimethyl-4,7-diphenyl-1,10-phenanthroline (BCP), 1,3,5-tri(phenyl-2-benzimidazolyl)benzene (TPBI), preferably PCBM, HAT-CN, C60, C70, PC70BM. PCBM- like C60 being a fullerene derivatives means [6,6]-phenyl C₆₁ butyric acid methyl ester.

The n-type semiconductor as defined above may be used as an electron transport material or hole blocking material for hole blocking layer or ETL and may comprise one, or two or more n-type metal oxide layers being compact or porous, if two or more layer are present, each n-type metal oxide layer is of a different or identical n-type metal oxide or one or more layers are an n-type semiconducting polymer. The blocking layer preferably hinders or prevents electron flow in the opposite direction and/or charge recombination.

According to one embodiment, the optoelectronic and/or photoelectrochemical device comprises a ETL comprising a n-type semiconducting polymer as defined above, which may be selected from n-type metal oxides semiconductor or from n-type semiconducting polymer.

In an embodiments, the n-type semiconductor layer or hole blocking layer/ETL is applied by one method selected from atomic layer deposition (ALD) and chemical bath deposition (CBD). Preferably, the metal oxide of n-type semiconducting layer is applied by CBD.

According to a further embodiment, the optoelectronic and/or photoelectrochemical device comprises an optional n-type surface-increasing structure or mesoporous ETL or surface-increasing scaffold structure, which provides an optional mesostructure in the solid state photovoltaic device. The n-type surface-increasing structure comprises one or more mesoporous metal oxide layers, each layer comprising a different or identical metal oxide being selected from metal oxide as defined for the n-type semiconductor or n-type semiconducting layer or conductive anode, such as SiO₂, TiO₂, SnO₂, Fe₂O₃, ZnO, WO₃, Nb₂O₅, MoO₃, NiO, SrTiO₃, ZrO₂, and combinations thereof. According to a preferred embodiment, the mesostructure comprises two films of metal oxide selected from TiO₂, ZrO₂ or three films of metal oxide selected from TiO₂, NiO, ZrO₂. Said mesostructure may be prepared by screen printing or spin coating, for example as is conventional for the preparation of porous semiconductor (e.g. TiO₂) layers in DSCs. Nanoporous semiconductor scaffold structures and layers have been disclosed, for example, in EP 0333641 and EP 0606453.

According to a further embodiment, the optoelectronic and/or photoelectrochemical device comprises an optional n-type surface-increasing structure made from non-conducting materials e.g. from an insulating material. In this case, the sensitizer or light-harvester, for example organic inorganic perovskite pigments, which are deposited on the surface increasing scaffold structure, are also in contact with an n-type semiconductor layer. In this case, the surface increasing structure does not continuously cover the n-type semiconductor layer. The surface-increasing structure is preferably structured on the nanoscale and is preferably a mesoporous structure. The structure of said surface increasing structure increase the effective surface compared to the surface of the solar cell.

The nanoparticles comprised in the surface increasing structure preferably have average dimensions and/or sizes in the range of 2 to 300 nm, preferably 3 to 200 nm, even more preferably 5 to 150 nm, and most preferably 5 to 100 nm. "Dimension" or "size" with respect to the nanoparticles means here extensions in any direction of space, preferably the average maximum extension of the nanoparticles.

The teachings herein are not intended to exclude the possibility that there are one or more intermediate layers between the scaffold structure and the conducting current collector support. Such intermediate layers, if present, would preferably be conducting or semiconducting.

According to another embodiment, the optoelectronic and/or photoelectrochemical device comprises a sensitizer layer or a light-harvester layer.

According to an embodiment, the sensitizer layer or a light-harvester layer of the photoelectrochemical and/or optoelectronic device comprises at least one pigment being selecting from organic, inorganic, organometallic and organic-inorganic pigments or a combination thereof. The thickness of said layer is in the range of 20 to 400 nm, 25 to 300 nm, or 100 to 300 nm, preferably 25 to 400 nm. The sensitizer is preferably a light absorbing compound or material. Preferably, the sensitizer is a pigment, and most preferably the sensitizer is an organic-inorganic pigment. A suitable sensitizer may be chosen from those known in the art.

The term "perovskite", for the purposes of this specification, refers to the "perovskite structure" and not specifically to the perovskite material, CaTiO₃. For the purposes of this specification, "perovskite" encompasses and preferably relates to any material that has the same type of crystal structure as calcium titanium oxide and of materials in which the bivalent cation is replaced by two separate monovalent cations. The perovskite structure has the general stoichiometry AMX₃, where "A" and "M" are cations and "X" is an anion. The "A" and "M" cations can have a variety of charges as in the original Perovskite mineral (CaTiO₃), the A cation is divalent and the M cation is tetravalent. Further, the perovskite formulae include structures having three or four anions, which may be the same or different, and/or one or two organic cations, and/or metal atoms carrying two or three positive charges, in accordance with the formulae presented elsewhere in this specification.

According to an embodiment, the photovoltaic device of the invention comprises one or more layers of an organic-inorganic perovskite. Said organic-inorganic perovskite may be selected from organic-inorganic perovskite described in WO 2014/020499A1, WO 2015/001459A1, WO 2015/107454A1, WO 2015/114521A1, PCT/IB2017/051538, EP16180656.7.

In an embodiment, the sensitizer layer comprises an organic-inorganic perovskite or a metal halide perovskite according to any one of perovskite-structures of formulae (II), (Ila), (IIb), (IIc), (IId), (IIe), (IIf) and/or (IIg) below:

AA'MX₄ (II)

AMX₃ (IIa)

AA'N_{2/3}X₄ (IIb)

AN_{2/3}X₃ (lIc)

BN_{2/3}X₄ (IId)

BMX₄ (IIe)

AA'A₁MX₃ (IIf)

AA₁MX₃ (IIg)

wherein,
- A and A' are organic, monovalent cations being independently selected from primary, secondary, tertiary or quaternary organic ammonium compounds, including N-containing hetero-rings and ring systems, A and A' having independently from 1 to 60 carbons and 1 to 20 heteroatoms;
- A₁ is an inorganic cation selected from Cs⁺, Rb⁺, and K⁺, preferably Cs⁺;
- B is an organic, bivalent cation selected from primary, secondary, tertiary or quaternary organic ammonium compounds having from 1 to 60 carbons and 2-20 heteroatoms and having two positively charged nitrogen atoms;
- M is selected from Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, Yb²⁺, [SnᵢPb₍₁₋ᵢ₎]⁺, [SnⱼGe₍₁₋ⱼ₎]⁺, and [PbₖGe₍₁₋ₖ₎]⁺, i, j and k being a number between 0.0 and 1.0;
- N is selected from the group of Bi³⁺ and Sb³⁺; and,
- X is independently selected from Cl⁻, Br⁻, I⁻, NCS⁻, CN⁻, NCO⁻, [I₍₃₋ₘ₎Clₘ]⁻, [I₍₃₋ₙ₎Brₙ]⁻, and [Br₍₃₋ᵤ₎Clᵤ]⁻; wherein m, n, u are a number between 0.0 and 3.0.

In one example, three of four possible X may be identical or different. For example, AMX₃ (formula IIa) may be expressed as formula (IIa'): AMXiXiiXiii (IIa'), wherein Xi, Xii, Xiii are independently selected from Cl⁻, Br⁻, I⁻, NCS⁻, CN⁻, NCO⁻, [I₍₃₋ₘ₎Clₘ]⁻, [I₍₃₋ₙ₎Brₙ]⁻, and [Br₍₃₋ᵤ₎Clᵤ]⁻; wherein m, n, u are a number between 0.0 and 3.0. A and M are as defined elsewhere in this specification, Xi, Xii, Xiii may thus be the same or different in this case. Similarly, formulae (IIc), (IIf), (IIg) may be expressed as (IIc'), (IIf'), (IIf') where X is Xi, Xii, and Xiii.

In another example, BMX₄ (formula IIe) may be expressed as formula (IIe'): AMXiXiiXiiiXiv (IIa'), wherein Xi, Xii, Xiii, and Xiv are independently selected from Cl⁻, Br⁻, I⁻, NCS⁻, CN⁻, NCO⁻, [I₍₃₋ₘ₎Clₘ]⁻, [I₍₃₋ₙ₎Brₙ]⁻, and [Br₍₃₋ᵤ₎Clᵤ]⁻; wherein m, n, u are a number between 0.0 and 3.0. Similarly, formulae (II), (lib), (IId) may be expressed as (II'), (IIb'), (IId') where X is Xi, Xii, Xiii, and Xiv.

Preferably, if X is Xi, Xii, Xii in formulae (IIa), (IIc), (IIf) and (IIg) or Xi, Xii, Xiii, Xiv in formulae (II), (Ilb), (IId) or (IIe), there are not more than two different anions.

According to the perovskite-structure of formula (IIf) or (IIg), A and A' are independently selected from methylammonium cation, formamidinium cations, iodo-carbamimidoyl cation or a combination of said cations.

According to a preferred embodiment, said organic-inorganic perovskite or metal halide perovskite comprises a perovskite-structure according to any one of the formulae (IIh) to (IIm):

APbX₃ (IIh)

ASnX₃ (IIi)

ABiX₄ (IIj)

AA'PbX₄ (IA)

AA'SnX₄ (IIj)

BPbX₄ (IL)

BSnX₄ (IIm)

wherein, A, A', B and X are as defined above in this specification. Preferably, X is preferably selected from Cl⁻, Br⁻ and I⁻, most preferably X is I⁻ or a mixture of Br⁻ and I⁻.

The sensitizer layer comprising organic-inorganic perovskite or metal halide perovskite may comprise a perovskite-structure according to any of the formulae (IIh) to (IIm), more preferably (IIh) and/or (IIi).

According to an embodiment, A and A' are monovalent cations of (IIh) thru (IIm) are selected independently from any one of the compounds of formulae (17) to (26) below: 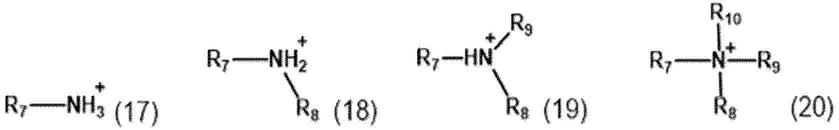 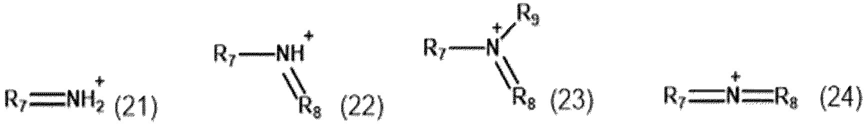 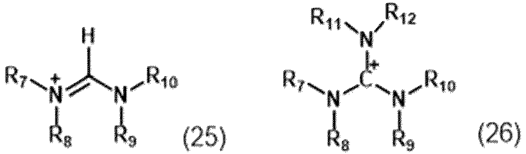 wherein, R₇, R₈, R₉, R₁₀, R₁₁ and R₁₂ are independently selected from H, C1-C15 organic substituents comprising from 0 to 15 heteroatoms. Counter anion of anyone of formulae (17) to (26), if applicable, may be selected from Cl⁻, Br⁻ or I⁻. According to an embodiment of said C1-C15 organic substituent any one, several or all hydrogens in said substituent may be replaced by halogen and said organic substituent may comprise up to fifteen N, S or O heteroatoms, and wherein, in any one of the compounds (17) to (26), the two or more of substituents present (R₇, R₈, R₉, R₁₀, R₁₁ and R₁₂, as applicable) may be covalently connected to each other to form a substituted or unsubstituted ring or ring system. Preferably, in a chain of atoms of said C1-C15 organic substituent, any heteroatom is connected to at least one carbon atom. Preferably, neighboring heteroatoms are absent and/or heteroatom-heteroatom bonds are absent in said C1-C15 organic substituent comprising from 0 to 15 heteroatoms. The heteroatoms may be selected from N, S, and/or O. According to one example, R₇, R₈, R₉, R₁₀, R₁₁ and R₁₂ are independently selected from H, C1 to C15 aliphatic and C4 to C15 aromatic or heteroaromatic substituents, wherein any one, several or all hydrogens in said substituent may be replaced by halogen and wherein, in any one of the compounds (17) to (26), the two or more of the substituents present may be covalently connected to each other to form a substituted or unsubstituted ring or ring system.

According to a preferred embodiment, the organic-inorganic perovskite is selected from a compound of formula (II), (IIa), (IIf) or (IIg).

According to an embodiment, B is bivalent cation selected from any one of the compounds of formulae (27) and (28) below:

R₁₃-G-R₁₄ (27)

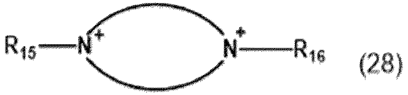

wherein, in the compound of formula (27), G is an organic linker structure having 1 to 10 carbons and 0 to 5 heteroatoms selected from N, S, and/or O, wherein one or more hydrogen atoms in said G may be replaced by halogen; wherein R₁₃ and R₁₄ are independently selected from a compounds of any one of formulae (17) to (26); and wherein, in the compound of formula (28), the circle containing said two positively charged nitrogen atoms represents a substituted or unsubstituted aromatic ring or ring system comprising 4 to 15 carbon atoms and 2 to 7 heteroatoms or 4 to 10 carbon atoms and 2 to 5 heteroatoms, wherein said nitrogen atoms are ring heteroatoms of said ring or ring system, and wherein the remaining of said heteroatoms may be selected independently from N, O and S and wherein R₁₅ and R₁₆ are independently selected from H and from a compounds of any one of formulae (17) to (26). Halogen atom substituting hydrogen atom totally or partially may also be present in addition to and/or independently of said 2 to heteroatoms.

According to an embodiment, R₇, R₈, R₉, R₁₀, R₁₁ and R₁₂ are independently selected from H, C1 to C10 alkyl, C2 to C10 alkenyl, C2 to C10 alkynyl, C4 to C10 heteroaryl and C6 to C10 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R₇, R₈, R₉, R₁₀, R₁₁ and R₁₂ may be replaced by halogen.

A, A' and B may be selected from the exemplary rings or ring systems of formulae (29) to (30) (for A, A') and from (31) to (33) (for B) below: 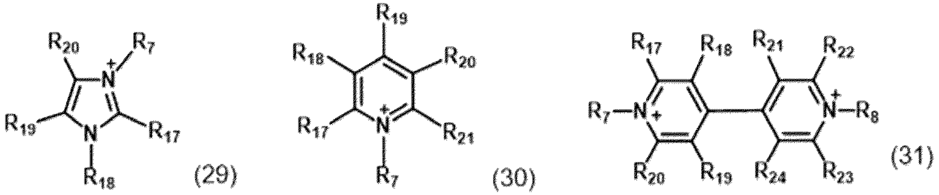 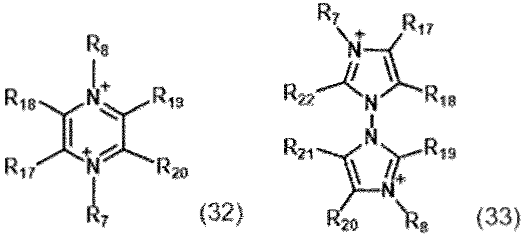 wherein
R₇ and R₈ are selected from substituents as defined above, and R₁₇, R₁₈, R₁₉, R₂₀, R₂₁, R₂₂, R₂₃ and R ₂₄ are independently selected from H, halogen and substituents as defined above for R₇, R₈, R₉, R₁₀, R₁₁ and R₁₂. Preferably, R₁₇, R₁₈, R₁₉, R₂₀, R₂₁, R₂₂, R₂₃ and R ₂₄ are selected from H and halogen, most preferably H.

According to a preferred embodiment, the metal M is selected from Sn²⁺ and Pb²⁺, preferably Pb²⁺. According to a preferred embodiment, N is Sb³⁺.

According to a preferred embodiment, the three or four X are independently selected from Cl⁻, Br⁻ and I⁻.

The counter electrode and/or metal layer and/or back contact generally comprises a catalytically active material, suitable to provide electrons and/or fill holes towards the inside of the device. The back contact may comprise one or more materials selected from Pt, Au, Ni, Cu, Ag, In, Ru, Pd, Rh, Ir, Os, porous carbon (C), conductive polymer and a combination of two or more of the aforementioned. Conductive polymers may be selected from polymers comprising poly(3,4-ethylenedioxythiophene):poly(styrenesulfonate) (PEDOT:PSS), polyaniline, polypyrrole, polythiophene, polybenzene, polyethylenedioxythiophene, polypropylenedioxy-thiophene, polyacetylene, and combinations of two or more of the aforementioned, for example.

According to another embodiment, the optoelectronic and/or photoelectrochemical device comprises a hole transport layer, i.e. comprising a compound of formula (I) being crosslinked or polymerized and forming a film. The hole transport layer is directly or indirectly in electric contact with the sensitizer layer and the positive electrode, i.e. the hole transport layer is between the sensitizer layer and the positive electrode. The positive electrode may be on the side exposed to light as is the case for the p-i-n perovskite solar cell illustrated on Fig. 1c or the "dark" side as it is the case for the n-i-p perovskite solar cell illustrated on Fig. 1a,b. The hole transport layer may include further dopants. However, the hole transport layer in the optoelectronic and/or photoelectrochemical device is free from solvent or other additives.

The hole transport layer HTL comprises a compound of formula (I) as defined herein and for the hole transport layer in the illustrated optoelectronic and/or photoelectrochemical device of Fig. 1.

According to one embodiment, the hole transport layer may comprise a further hole transporting material or further layers of hole conductive material different from the hole transporting material, which may be applied on or before the hole transport layer. The further hole conductive material may be selected from poly(3,4-ethylenedioxythiophene):poly(styrenesulfonate) (PEDOT:PSS), poly(3,4-ethylenedioxythiophene): poly(styrenesulfonate):grapheme nanocomposite (PEDOT:PSS:graphene), poly(N-vinylcarbazole) (PVK) and sulfonated poly(diphenylamine) (SPDPA), preferably from PEDOT:PSS, PEDOT:PSS:graphene and PVK, more preferably from PEDOT:PSS. Conductive polymers may also be selected from polymers comprising polyaniline, polypyrrole, polythiophene, polybenzene, polyethylenedioxythiophene, polypropylenedioxy-thiophene, polyacetylene, and combinations of two or more of the aforementioned.

According to another embodiment, the optoelectronic and/or photoelectrochemical device comprises a conducting current providing layer or metallic contact forming the counter electrode. The conducting current providing layer and/or metallic contact and/or back contact comprises a catalytically active material, suitable to provide electrons and/or fill holes towards the inside of the device. The conducting current providing layer and/or metallic contact and/or back contact may comprise one or more materials selected from Pt, Au, Ni, Cu, Ag, In, Ru, Pd, Rh, Ir, Os, porous carbon (C), conductive polymer and a combination of two or more of the aforementioned. Conductive polymers may be selected from polymers comprising poly(3,4-ethylenedioxythiophene):poly(styrenesulfonate) (PEDOT:PSS), polyaniline, polypyrrole, polythiophene, polybenzene, polyethylenedioxythiophene, polypropylenedioxy-thiophene, polyacetylene, and combinations of two or more of the aforementioned, for example.

In a further embodiment, the optoelectronic and/or photoelectrochemical device comprises a layer underlying the hole transport layer being selected from a hole injection layer, a sensitizer layer, a light-harvester layer, or a conducting current collector, wherein said underlying layer is not crosslinked. Said underlying layer may be the layer under the hole transport layer or the under layer in electric contact directly with the hole transporting layer or indirectly with the hole transporting layer through an optional layer. The optional layer may be layer blocking a specific type of charge (electron or hole) to avoid any recombination, concentrating the charge or facilitating the flow of a specific charge. The selection of the optional layer depends on the direction of the flow of the electrons or the holes between the two opposite side and their respective polarity, and/or the energy level of the preceding layer of the underlying layer.

According to another embodiment, the optoelectronic and/or photoelectrochemical device comprises a layer overlying the hole transport layer, said layer being selected from an emissive layer when the underlying layer is a hole injection layer, from a counter electrode or a conducting current providing layer when the underlying layer is a sensitizer layer or a light-harvesting layer, or from a sensitizer layer when the underlying layer is a conducting current collector. Said overlying layer may be the layer above the hole transport layer or the above layer in electric contact directly with the hole transporting layer or indirectly with the hole transporting layer through an optional layer. The optional layer may be layer blocking a type of charge (electron or hole), concentrating the charge or facilitating the flow of a specific charge. The selection of the optional layer depends on the direction of the flow of the electrons or the holes between the two opposite sides and their respective polarity, and/or the energy level of the preceding layer of the underlying layer.

One or more layers may compose the layers overlying or underlying the hole transport layer. Due to the mechanical properties of the film formed by the polymerization of the hole transporting material as defined in formula (I), the underlying and/or overlying layers are protected against mixing with the further applied layers during their application by liquid process, against UV light and humidity/moisture, which improves the stability of the device.

### Fabrication of an optoelectronic and/or photoelectrochemical device

In further aspect, the present teachings provide a method for fabricating an optoelectronic and/or photoelectrochemical device comprising a hole transport layer, said method comprising
- providing a first layer, said layer being an underlying layer;
- providing a second layer, wherein the second layer is the hole transport layer, onto the underlying layer by liquid deposition, said steps of providing being
   - applying a hole transporting material comprising the compound of formula **(I)** by liquid process onto the underlying layer,
   - crosslinking the hole transporting material by thermal, chemical or irradiative means; and
- providing a third layer, said layer being an overlying layer;
wherein the underlying layer is selected from a hole injection layer, a sensitizer layer, a light-harvester layer, or a conducting current collector; wherein the overlying layer is selected from an emissive layer when the underlying layer is a hole injection layer, from a conducting current providing layer when the underlying layer is a sensitizer layer or a light-harvesting layer, or from a sensitizer layer when the underlying layer is a conducting current collector. The liquid process to apply the polymerizable compound of formula **(I)** is selected from spin coating, blade coating, meniscus coating, printing, or spray deposition. The polymerizable compound of formula (I) is preferably in the form of a liquid or a liquid composition.

The method for fabricating an optoelectronic and/or photoelectrochemical device further comprises steps of providing additional layers underlying said first underlying layer and/or providing additional layers overlying said third, overlying layer to form a specific optoelectronic and/or photoelectrochemical device, said additional layer being selected from substrate layer, conductive anode, ETL, EIL, conductive cathode or metallic contact.

The means of crosslinking the hole transporting material is selected from thermal means such as providing a heating source of temperature up to 260°C or up to 110 °C when a crosslinking agent with two or more thiol groups are present, by chemical or irradiative means such as providing chemical inducer, e.g. azoisobutyronitrile, or by irradiative means by providing UV photoirradiation.

According to one embodiment of the method, the underlying layer is not crosslinked or is not further submitted to crosslinking before applying the hole transport layer. The underlying layer may be annealed, e.g. to produce the sensitizer but crosslinking of the underlying layer is not mandatory or required for or before the application of the hole transport layer. The polymerization *in-situ* of the hole transport material comprising the polymerizable compound of formula **(I)** stabilizes the underlying layer in the device against environmental harms (UV light, humidity, heat) and harms ( such as layer mixing) during the fabrication process by liquid method.

The crosslinked hole transporting material in the hole transport layer forms a homogenous polymer film, filling the pores of the underlying layer, wherein the hole transport layer does not mix with the liquid material of the overlying layer during the application of said overlaying layer.

In another embodiment, the optoelectronic and/or photoelectrochemical device obtained by the method is an organic light-emitting diode, the underlying layer is the hole injection layer and the overlaying layer is the emissive layer.

In a further embodiment, the optoelectronic and/or photoelectrochemical device obtained by the method is a solid state solar cell having the underlying layer being the sensitizer layer or light-harvesting layer and the overlying layer being the counter electrode or a conducting current providing layer or the underlying layer being a conducting current collector and the overlying layer being a sensitizer layer or a light-harvesting layer.

According to one embodiment, the optoelectronic and/or photoelectrochemical device obtained by the method is a solid state solar cell, wherein the sensitizer layer or the light-harvesting layer comprises an organic-inorganic perovskite.

The compounds, devices, and methods are described more concretely with reference to the following examples, which, are not intended to be limiting .

*General Synthesis Scheme of Compounds of General Formule **(I).***

Hole transporting compound **HTM1** containing 9,9'-spirobisfluorene moiety and cross-linkable vinyl groups corresponding to the general formula **(I)** was prepared by the four-step synthesis route shown in Scheme 1. The first step was the Buchwald-Hartwig C-N cross coupling reaction of commercially available 2-(4-bromophenyl)-1,3-dioxolane (Sigma-Aldrich) with *p*-anisidine (Sigma-Aldrich) to form the diphenylamine derivative **1** of Scheme 1. Intermediate **1** was used in the Buchwald-Hartwig C-N cross coupling reaction with 2,2',7,7'-tetrabromo-9,9'-spirobifluorene (Sigma-Aldrich) to obtain the intermediate **2,** which in turn was transformed to the 9,9-spirobisfluorene derivative **3,** possessing aldehyde groups. The last step was the Wittig rection that provided the target compound **HTM1.**

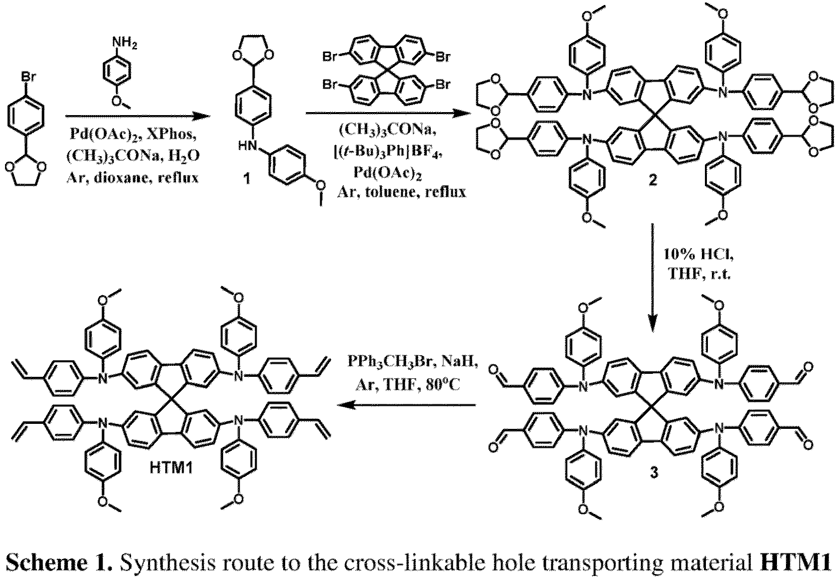

Hole transporting compound **HTM2** containing 9,9'-spirobisfluorene moiety and cross-linkable vinyl groups corresponding to the general formula **(I)** was prepared by the two-step synthesis route shown in Scheme 2. The first step was the Buchwald-Hartwig C-N cross coupling reaction of commercially available 2,2',7,7'-tetrabromo-9,9'-spirobifluorene (Sigma-Aldrich) with p-anisidine (Sigma-Aldrich). The intermediate **4** was alkylated with 4-(chloromethyl)styrene to isolate the target compound **HTM2.**

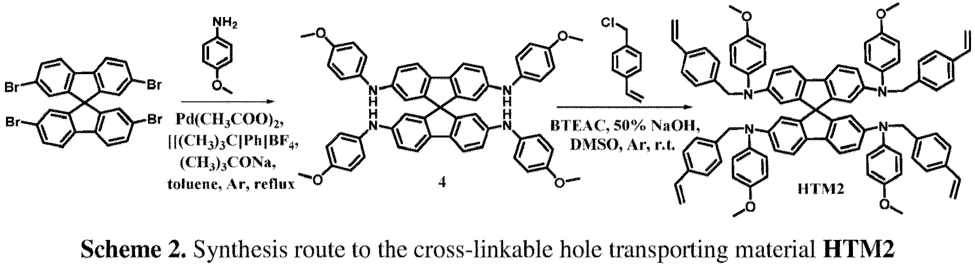

Hole transporting compound **HTM3** containing 9,9'-spirobisfluorene moiety and cross-linkable vinyl groups corresponding to the general formula **(I)** was prepared by the four-step synthesis route shown in Scheme 3. The first step was alkylation of 4-bromophenol with 1,2-dibromoethane (Sigma-Aldrich). The resulting intermediate **5** was transformed to the vinyl derivative **6.** It was used in C-N cross coupling reaction with p-anisidine (Sigma-Aldrich) provided the diphenylamine derivative **7.** The last step was the Buchwald-Hartwig C-N cross coupling reaction of commercially available 2,2',7,7'-tetrabromo-9,9'-spirobifluorene (Sigma-Aldrich) with diphenylamine compound **7** to obtain the target compound **HTM3.**

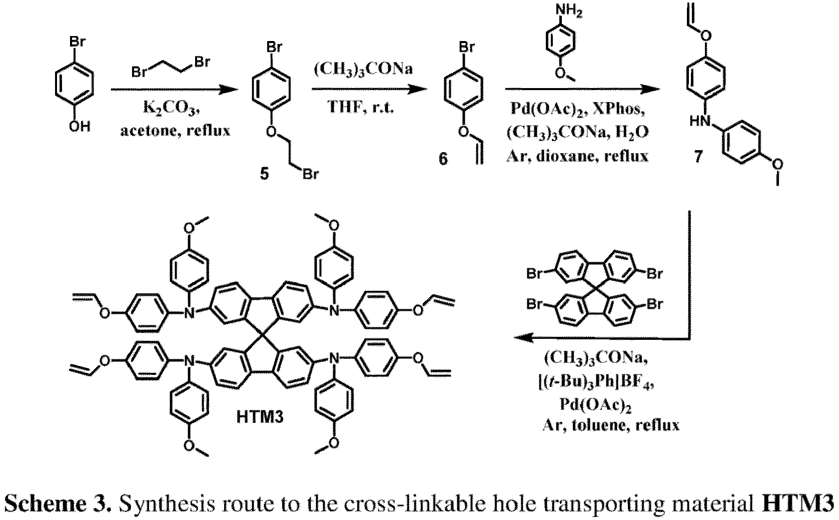

Hole transporting compound **HTM4** containing 9,9'-spirobisfluorene moiety and cross-linkable vinyl groups corresponding to the general formula **(I)** was prepared by the three-step synthesis route shown in Scheme 4. The first step was alkylation of 3-bromo-9H-carbazole with 4-vinylbenzyl chloride (Sigma-Aldrich). The resulting intermediate 8 was reacted with p-anisidine (Sigma-Aldrich) to obtain derivative **9,** which was further used in Buchwald-Hartwig C-N cross coupling reaction with commercially available 2,2',7,7'-tetrabromo-9,9'-spirobifluorene (Sigma-Aldrich) to obtain the target compound **HTM4.**

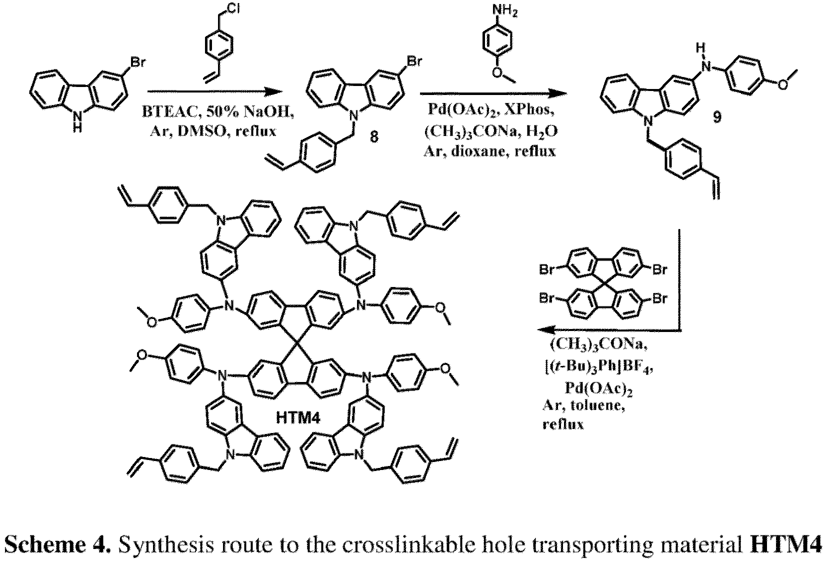

### General Preparatory Scheme for Perovskite Solar Cells

As substrate for the devices, etched fluorine/indium-doped tin oxide (FTO/ITO) as anode is used and is cleaned prior to assembly. The cleaned substrate is then deposited with an ETM or HTM depending on the architecture. The remaining steps are performed under nitrogen conditions. A perovskite precursor solution is prepared using standard stock solutions in DMSO/DMF then spin coated onto the substrate. The resulting perovskite film is annealed at 100 °C. The perovskite layer is then covered with a ETM or HTM depending on the selected architecture. Cathode electrode (Ag, Au, or Al) is then deposited by thermal evaporation. Fig. 1 shows a cross-sectional view of the resulting photovoltaic cells with different architectures.

### Examples

Information on examples of real embodiments is provided below, describing the modes of preparation compounds (**HTM1-HTM4**) and properties thereof. This information is provided for illustrative purpose and is not limiting.

### Synthesis of the intermediates 1-9

### N-[4-(1,3-dioxolan-2-yl)phenyl]-4-methoxyaniline (1):

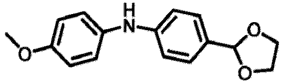

Anhydrous dioxane (25 mL) with few drops of distilled water (0.02 eq) was purged with argon for 20 minutes. After that, the temperature was raised to 80 °C, palladium (II) acetate (0.005 eq) and XPhos (0.015 eq) were added. The mixture was stirred for 1.5 minutes, and the temperature was raised to 110 °C. 2-(4-Bromophenyl)-1,3-dioxolane (5 g, 21.8 mmol, 1 eq), p-Anisidine (3.2 g, 26.2 mmol, 1.2 eq) and sodium tert-butoxide (1.4 eq) were added and the solution was refluxed for 25 minutes. After cooling to room temperature, the reaction mixture was extracted with ethyl acetate and distilled water. The organic layer was dried over anhydrous Na₂SO₄, filtered and the solvent evaporated. The crude product was purified by column chromatography using 1:1:2.5:20.5 v/v/v/v/v diethyl ether/ethyl acetate/THF/n-hexane as an eluent. Pale yellow crystals were collected as a final product. (4.5 g, 76.2 %). ¹H NMR (400 MHz, THF-*d*₆) δ 7.16 (d, *J =* 7.6 Hz, 2H), 7.12 - 6.95 (m, 3H), 6.91 - 6.77 (m, 4H), 5.59 (s, 1H), 4.07 - 3.95 (m, 2H), 3.95 - 3.81 (m, 2H), 3.74 (s, 3H). ¹³C NMR (101 MHz, THF) δ 153.21, 144.60, 134.30, 126.62, 125.57, 122.00, 119.51, 112.34, 102.08, 62.89, 52.73 ppm. Anal. calculated, for: C₁₆H₁₇NO₃: C, 70.83; H, 6.32; N, 5.16; found: C, 70.94; H, 6.36; N, 5.12. C₁₆H₁₇NO₃[M⁺] exact mass = 271.12, MS (ESI) = 272.17.

### N²,N^{2'},N⁷,N^{7'}-tetrakis[4-(1,3-dioxolan-2-yl)phenyl]-N²,N^{2'},N⁷,N^{7'}-tetrakis(4-methoxyphenyl)-9,9'-spirobi(fluorene)-2,2',7,7'-tetraamine (2):

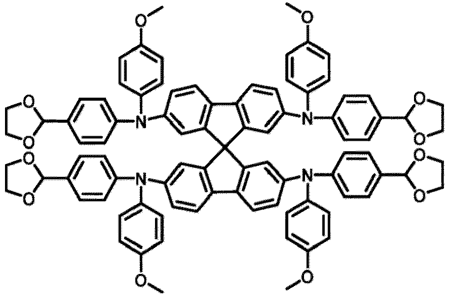

A solution of 2,2',7,7'-tetrabromo-9,9'-spirobifluorene (0.58 g, 0.9 mmol, 1 eq) and compound **1** (1.5 g, 5.5 mmol, 6 eq) in anhydrous toluene (13 mL) was purged with argon for 30 minutes. Afterwards, palladium (II) acetate (0.02 eq), tri-tert-butylphosphonium tetrafluoroborate (0.027 eq) and sodium tert-butoxide (6 eq) were added and the solution was refluxed under argon atmosphere for 18 hours. After cooling to room temperature, the reaction mixture was filtered through celite, extracted with ethyl acetate and distilled water. The organic layer was dried over anhydrous Na₂SO₄, filtered and solvent evaporated. The crude product was purified by column chromatography using 11:14 v/v THF/n-hexane as an eluent. Pale green solid was collected as an intermediate product 2. (0.75 g, 59.2%). ¹H NMR (400 MHz, THF-*d*₆) δ 7.50 (d, *J =* 8.0 Hz, 4H), 7.22 (d, *J* = 8.0 Hz, 8H), 6.97 (d, *J* = 8.4 Hz, 8H), 6.91 - 6.76 (m, 20H), 6.65 (s, 4H), 5.62 (s, 4H), 4.07 - 3.95 (m, 8H), 3.94 - 3.85 (m, 8H), 3.75 (s, 12H). ¹³C NMR (101 MHz, THF) δ 154.64, 148.15, 147.00, 145.08, 138.59, 134.56, 129.34, 125.31, 124.86, 122.41, 118.60, 118.36, 117.33, 112.70, 101.64, 62.99, 52.77, 34.53 ppm. Anal. calcd for C₈₉H₇₆N₄O₁₂: C, 76.71; H, 5.50; N, 4.02; found: C, 76.47; H, 5.52; N, 4.01.

### 4,4',4",4‴-(9,9'-spirobi[fluorene]-2,2',7,7'-tetrayltetrakis{(4-methoxyphenyl)azanediyl}) tetrabenzaldehyde (3):

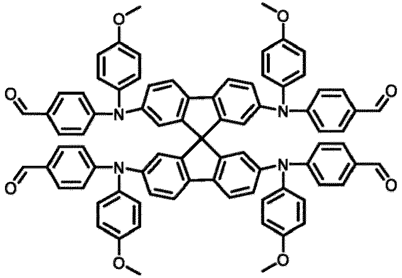

To a solution of compound 2 (1.3 g, 0.9 mmol, 1 eq) in THF (14 mL) was dropped 3 mL 10% HCl solution and reaction mixture stirred for 20 minutes. The reaction mixture was extracted with ethyl acetate and distilled water. The organic layer was dried over anhydrous Na₂SO₄, filtered and the solvent evaporated. The crude product was recrystallized from 2:1 v/v THF/ethanol gave as yellow **3** crystals. (0.92 g, 81.4 %). ¹H NMR (400 MHz, THF-*d*₆) δ 9.74 (s, 4H), 7.70 (d, *J=* 8.0 Hz, 4H), 7.60 (d, *J=* 8.4 Hz, 8H), 7.14 - 6.91 (m, 20H), 6.91 - 6.77 (m, 12H), 3.80 (s, 12H). ¹³C NMR (101 MHz, THF) δ 186.95, 156.05, 151.39, 148.04, 144.10, 136.71, 136.15, 128.73, 127.23, 126.18, 124.70, 119.43, 119.08, 115.63, 113.26, 63.68, 53.00 ppm. Anal. calculated for C₈₁H₆₀N₄O₈: C, 79.92; H, 4.97; N, 4.60; found: C, 79.57; H, 4.92; N, 4.61.

### N²,N^{2'},N⁷,N^{7'}-tetrakis(4-methoxyphenyl)-9,9'-spirobi[fluorene]-2,2',7,7'-tetraamine (4):

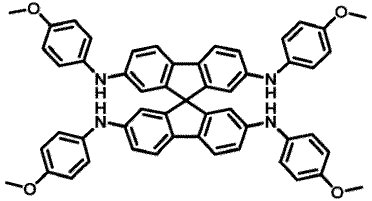

A solution of compound 2,2',7,7'-tetrabromo-9,9'-spirobifluorene (3 g, 4.7 mmol, 1 eq) and p-anisidine (5.9 g, 47.5 mmol, 10 eq) in anhydrous toluene (47 mL) was purged with argon for 30 minutes. Afterwards, palladium (II) acetate (0.02 eq), tri-tert-butylphosphonium tetrafluoroborate (0.027 eq) and sodium tert-butoxide (8 eq) were added and the solution was refluxed under argon atmosphere for 28 hours. After cooling to room temperature, the reaction mixture was extracted with ethyl acetate and distilled water. The organic layer was dried over anhydrous Na₂SO₄, filtered and solvent evaporated. The crude product was purified by column chromatography using 8:17 v/v THF/n-hexane as an eluent. Pale green solid were collected as a final product (2.7 g, 71.1 %). ¹H NMR (400 MHz, DMSO-*d*₆) δ 7.73 (s, 4H), 7.54 (d, *J=* 8.0 Hz, 4H), 7.00 - 6.87 (m, 12H), 6.79 (d, *J=* 8.4 Hz, 8H), 6.23 (s, 4H), 3.67 (s, 12H). ¹³C NMR (101 MHz, DMSO) δ 154.14, 150.79, 144.28, 136.61, 132.96, 120.61, 119.86, 114.96, 113.37, 111.64, 65.48, 55.65 ppm. Anal. calculated for C₅₃H₄₄N₄O₄: C, 79.48; H, 5.54; N, 7.00; found: C, 79.77; H, 5.52; N, 7.04.

### 1-Bromo-4-(2-bromoethoxy)benzene (5):

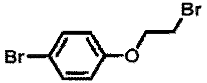

To a solution of 4-bromophenol (10 g, 57.8 mmol, 1 eq) and K₂CO₃ (2.2 eq) in acetone (100 mL) was added 1,2-dibromoethane (50 g, 266.2 mmol, 4.6 eq) and refluxed for three days. After cooling to room temperature, the reaction mixture was extracted with ethyl acetate and distilled water. The organic layer was dried over anhydrous Na₂SO₄, filtered and solvent evaporated. The crude product was crystallized from ethanol (40 mL). The obtained crystals were filtered off and washed with hot ethanol three times to collect intermediate **5** as a light creamy colour crystals. (12.40 g, 76.7 %). The NMR spectra were identical to the corresponding spectra of the product referred in [A. Gegout, J. L. Delgado and et. al, New J. Chem., 2009, 33, 2174].

### 1-Bromo-4-(vinyloxy)benzene (6):

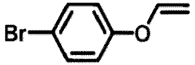

The solution of compound **5** (5 g, 17.9 mmol, 1 eq) and sodium tert-butoxide (1.6 eq) in THF (15 mL) was stirred overnight. The reaction mixture was filtered, and the organic solvent was removed by vacuum. The crude product was purified by column chromatography using n-hexane as an eluent. The colorless oil was collected as a final product **6** (2.74 g, 77.1 %). ¹H NMR (400 MHz, DMSO-*d*₆) δ 7.53 (d, *J* = 8.8 Hz, 2H), 7.03 (d, *J* = 8.8 Hz, 2H), 6.86 (2d, *J₁* = 17.6, *J₂* = 10.9 Hz, 1H), 4.77 (d, *J =* 17.6 Hz, 1H), 4.52 (d, *J =* 10.9 Hz, 1H). ¹³C NMR (101 MHz, DMSO) δ 155.95, 148.30, 133.04, 119.24, 115.16, 96.46 ppm. Anal. calcd for C₈H₇BrO: C, 48.27; H, 3.54; found: C, 48.47; H, 3.52.

### 4-Methoxy-N-[4-(vinyloxy)phenyl]aniline (7)

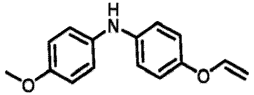

Anhydrous dioxane (14 mL) with few drops of distilled water (0.02 eq) was purged with argon for 20 minutes. After that, the temperature was raised to 80 °C, palladium (II) acetate (0.005 eq) and XPhos (0.015 eq) were added. The mixture was stirred for 1.5 minutes, and temperature was raised to 110 °C. Compound **6** (2.3 g, 11.6 mmol, 1 eq), p-anisidine (1.7 g, 13.9 mmol, 1.2 eq) and sodium tert-butoxide (1.4 eq) were added and stirred for 5 minutes. The reaction mixture was filtered, and the organic solvent was removed by vacuum. The crude product was purified by column chromatography using 1:9 v/v THF/n-hexane as an eluent. The orange oil was collected as a product **7** (2.48 g, 88.9 %). ¹H NMR (400 MHz, DMSO-*d*₆) δ 7.73 (s, 1H), 6.98 (d, *J=* 8.8 Hz, 2H), 6.95 - 6.87 (m, 4H), 6.84 (d, *J =* 8.8 Hz, 2H), 6.73 (2d, *J₁* = 17.6, *J₂* = 10.9 Hz, 1H), 4.57 (d, *J =* 17.6 Hz, 1H), 4.33 (d, *J=* 10.9 Hz, 1H), 3.68 (s, 3H). ¹³C NMR (101 MHz, DMSO) δ 153.93, 150.14, 149.21, 141.40, 137.33, 119.90, 118.57, 117.05, 115.03, 93.62, 55.68 ppm. Anal. calculated for C₁₅H₁₅NO₂: C, 74.67; H, 6.27; N, 5.81 found: C, 74.79; H, 6.22; N, 5.85.

### 3-Bromo-9-(4-vinylbenzyl)-9H-carbazole (8):

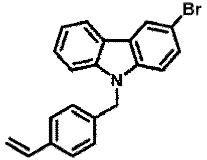

3-bromocarbazole (1 g, 4.1 mmol, 1 eq) was dissolved in dimethylsulfoxyde (25 mL) and purged with argon for 30 minutes. Afterwards, benzyltriethylammonium chloride (0.1 eq) and 50% NaOH (0.4 mL) solution were added. When the reaction mixture turned red 4-vinylbenzylchloride (0.63 mL, 4.5 mmol, 1.1 eq) was slowly dropped and the mixture was stirred at room temperature for 3 hours under argon atmosphere. The obtained product was filtered off and washed with ethanol to collect **8** as a white solid. (1.25 g, 85.0 %). The NMR spectra were identical to the corresponding spectra of the product referred in [S. Daskeviciute-Geguziene, A. Magomedov and et.al, Chem. Commun., 2022, 58, 7495].

### N-(4-methoxyphenyl)-9-(4-vinylbenzyl)-9H-carbazol-3-amine (9):

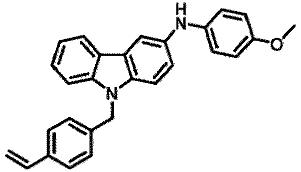

Anhydrous dioxane (10 mL) with few drops of distilled water (0.02 eq) was purged with argon for 20 minutes. After that, the temperature was raised to 80 °C, palladium (II) acetate (0.005 eq) and XPhos (0.015 eq) were added. The mixture was stirred for 1.5 minutes and temperature was raised to 110 °C. Compound **8** (2 g, 5.5 mmol, 1 eq), *p*-anisidine (0.8 g, 6.6 mmol, 1.2 eq) and sodium *tert*-butoxide (1.4 eq) were added and stirred for 5 minutes. After cooling to room temperature, reaction mixture was extracted with ethyl acetate and distilled water. The organic layer was dried over anhydrous Na₂SO₄, filtered and the solvent evaporated. The crude product was purified by column chromatography using 1:9 v/v THF/n-hexane as an eluent. Pale grey solid was collected as a product **9.** (1.74 g, 78.0 %). The NMR spectra were identical to the corresponding spectra of the product referred in [S. Daskeviciute-Geguziene, A. Magomedov and et.al, Chem. Commun., 2022, 58, 7495].

### Example 1

### N²,N^{2'},N⁷N^{7'}-tetrakis(4-methoxyphenyl)-N²,N^{2'},N⁷,N^{7'}-tetrakis(4-vinylphenyl)-9,9'-spirobi[fluorene]- 2,2',7,7'-tetraamine (see Scheme 1, HTM1):

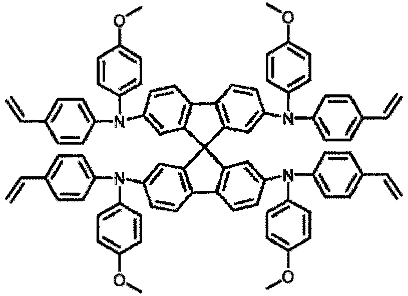

To a solution of PPh₃CH₃Br (4 equivalents) and NaH (4 equivalents) in dry THF (30 mL) was stirred at room temperature under argon atmosphere for 1 hour, followed by adding dry THF (15 mL) solution of the intermediate **3** (0.7 g, 0.6 mmol, 1 eq). The reaction mixture was heated and stirred at 80 °C for 22 hours. After cooling to room temperature, the solution was quenched by water and stirred for 5 minutes. The reaction mixture was filtered through celite, extracted with ethyl acetate and distilled water. The organic layer was dried over anhydrous Na₂SO₄, filtered and solvent evaporated. The crude product was purified by column chromatography using 6:19 v/v THF/n-hexane as an eluent. The obtained product was precipitated from THF into 15 times excess of ethanol. The precipitate was filtered off and washed with ethanol to collect **HTM1** as a pale yellow solid. (0.54 g, 77.5 %). ¹H NMR (400 MHz, THF-*d*₆) δ 7.51 (d, *J=* 8.4 Hz, 4H), 7.20 (d, *J=* 8.6 Hz, 8H), 6.97 (d, *J =* 8.6 Hz, 8H), 6.92 - 6.78 (m, 20H), 6.67 - 6.53 (m, 8H), 5.57 (d, *J =* 17.6 Hz, 4H), 5.05 (d, *J=* 10.9 Hz, 4H), 3.75 (s, 12H). ¹³C NMR (101 MHz, THF) δ 154.68, 148.14, 146.11, 144.97, 138.45, 134.54, 134.47, 128.93, 124.82, 124.81, 122.14, 119.29, 118.32, 117.12, 112.68, 108.59, 63.78, 52.76 ppm. Anal. calculated for C₈₅H₆₈N₄O₄: C, 84.41; H, 5.67; N, 4.63; found: C, 84.27; H, 5.62; N, 4.65. C₈₅H₆₈N₄O₄[M⁺] exact mass = 1208.52, MS (ESI) = 1209.44.

### Example 2

### N²,N^{2'},N⁷,N^{7'}-tetrakis(4-methoxyphenyl)-N²,N^{2'},N⁷,N^{7'}-tetrakis(4-vinylbenzyl)-9,9'-spirobi[fluorene]-2,2',7,7'-tetraamine (see Scheme 2, HTM2):

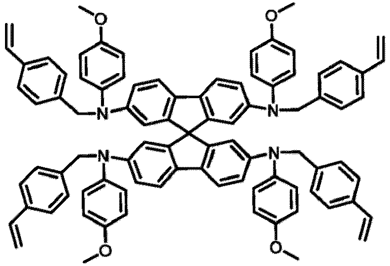

Compound **4** (0.8 g, 1.0 mmol, 1 equivalent) in dimethylsulfoxyde (35 mL) was dissolved and purged with argon for 30 minutes. Afterwards, benzyltriethylammonium chloride (0.4 equivalents) and 50% NaOH (0.4 mL) solution were added. The color of the reaction should turn black and then was slowly dropped 4-(chloromethyl)styrene (0.73 g, 4.8 mmol, 4.8 equivalents) under argon atmosphere and was stirred at room temperature for 24 hours. The reaction mixture was filtered through celite, extracted with ethyl acetate and distilled water. The organic layer was dried over anhydrous Na₂SO₄, filtered and solvent evaporated. The crude product was purified by column chromatography using 1:4 v/v acetone/n-hexane as an eluent. The obtained product was precipitated from acetone into 15 times excess of ethanol. The precipitate was filtered off and washed with ethanol to collect **HTM2** as a pale green solid. (0.91 g, 72.2 %). ¹H NMR (400 MHz, DMSO-*d*₆) δ 7.35 (d, *J=* 8.0 Hz, 4H), 7.28 (d, *J=* 7.6 Hz, 8H), 7.11 (d, *J=* 7.6 Hz, 8H), 6.85 (d, *J=* 8.0 Hz, 8H), 6.79 - 6.53 (m, 16H), 6.17 (s, 4H), 5.67 (d, *J=* 17.6 Hz, 4H), 5.18 (d, *J=* 10.9 Hz, 4H), 4.70 (s, 8H), 3.63 (s, 12H). ¹³C NMR (101 MHz, DMSO) δ 155.24, 150.15, 147.69, 140.89, 139.28, 136.78, 136.07, 133.35, 127.33, 126.55, 124.36, 120.29, 117.59, 114.91, 114.23, 112.97, 65.85, 56.02, 55.55 ppm. Anal. calculated for C₈₉H₇₆N₄O₄: C, 84.46; H, 6.05; N, 4.43; found: C, 84.57; H, 6.02; N, 4.44. C₈₉H₇₆N₄O₄[M⁺] exact mass = 1264.59, MS (ESI) = 1265.30.

### Example 3

### N²,N^{2'},N⁷,N^{7'}-tetrakis(4-methoxyphenyl)-N²,N^{2'},N⁷,N^{7'}-tetrakis[4-(vinyloxy)phenyl]-9,9'-spirobi[fluorene]-2,2',7,7'-tetraamine (see Scheme3, HTM3):

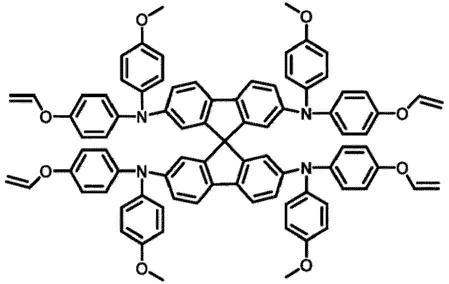

A solution of compound 2,2',7,7'-tetrabromo-9,9'-spirobifluorene (0.5 g, 0.8 mmol, 1 equivalent) and compound **7** (1.1 g, 4.7 mmol, 6 equivalents) in anhydrous toluene (10 mL) was purged with argon for 30 minutes. Afterwards, palladium (II) acetate (0.02 equivalents), tri-*tert-*butylphosphonium tetrafluoroborate (0.027 equivalents) and sodium tert-butoxide (6 equivalents) were added and the solution was refluxed under argon atmosphere for 16 hours. After cooling to room temperature, the reaction mixture was filtered through celite, extracted with ethyl acetate and distilled water. The organic layer was dried over anhydrous Na₂SO₄, filtered and solvent evaporated. The crude product was purified by column chromatography using 1:4 v/v THF/n-hexane as an eluent. The obtained product was precipitated from THF into 15 times excess of ethanol. The precipitate was filtered off and washed with ethanol to collect **HTM3** as a pale green solid. (0.45 g, 45.3 %). ¹H NMR (400 MHz, THF-*d*₆) δ 7.45 (d, *J =* 8.3 Hz, 4H), 6.96 - 6.87 (m, 16H), 6.87 - 6.77 (m, 20H), 6.68 (2d, *J₁* = 17.6, *J₂* = 10.9 Hz, 4H), 6.54 (s, 4H), 4.64 (d, *J =* 17.6 Hz, 4H), 4.33 (d, *J =* 10.9 Hz, 4H), 3.73 (s, 12H). ¹³C NMR (101 MHz, THF) δ 154.23, 150.03, 148.19, 146.94, 145.43, 141.98, 139.00, 133.85, 123.97, 122.13, 121.02, 118.04, 116.12, 115.63, 112.58, 91.34, 63.79, 52.76 ppm. Anal. calcd for C₈₅H₆₈N₄O₈: C, 80.17; H, 5.38; N, 4.40; found: C, 80.47; H, 5.39; N, 4.34. C₈₅H₆₈N₄O₈[M⁺] exact mass = 1272.50, MS (ESI) = 1272.96.

### Example 4

### N²,N^{2'},N⁷,N^{7'}-tetrakis(4-methoxyphenyl)-N²,N^{2'},N⁷,N^{7'}-tetrakis[9-(4-vinylbenzyl)-9H-carbazol-3-yl]-9,9'-spirobi[fluorene]-2,2',7,7'-tetraamine (see Scheme4, HTM4):

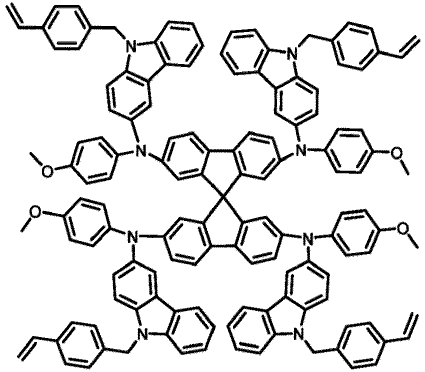

A solution of compound 2,2',7,7'-tetrabromo-9,9'-spirobifluorene (0.5 g, 0.8 mmol, 1 equivalents) and compound **9** (1.9 g, 4.7 mmol, 6 equivalents) in anhydrous toluene (15 mL) was purged with argon for 30 minutes. Afterwards, palladium (II) acetate (0.02 equivalents), tri-*tert-*butylphosphonium tetrafluoroborate (0.027 equivalents) and sodium *tert*-butoxide (6 equivalents) were added and the solution was refluxed under argon atmosphere for 18 hours. After cooling to room temperature, the reaction mixture was filtered through celite, extracted with ethyl acetate and distilled water. The organic layer was dried over anhydrous Na₂SO₄, filtered and solvent evaporated. The crude product was purified by column chromatography using 2:3 v/v THF/n-hexane as an eluent. The obtained product was precipitated from THF into 15 times excess of ethanol. The precipitate was filtered off and washed with ethanol to collect **HTM4** as a pale green solid. (0.98 g, 64.5 %). ¹H NMR (400 MHz, THF-*d*₆) δ 7.93 (d, *J=* 7.6 Hz, 4H), 7.82 (s, 4H), 7.44 - 7.24 (m, 24H), 7.21 - 7.04 (m, 16H), 6.96 (d, *J=* 8.8 Hz, 8H), 6.83 - 6.71 (m, 12H), 6.69 - 6.58 (m, 8H), 5.69 (d, *J=* 17.6 Hz, 4H), 5.53 (s, 8H), 5.12 (d, *J =* 10.9 Hz, 4H), 3.68 (s, 12H). ¹³C NMR (101 MHz, THF) δ 153.50, 148.25, 145.98, 140.15, 139.36, 138.70, 135.65, 135.53, 134.90, 134.64, 133.00, 124.88, 124.46, 123.70, 123.08, 122.43, 122.00, 120.96, 119.47, 118.48, 117.49, 116.88, 115.11, 114.75, 112.32, 110.88, 107.61, 107.01, 52.70, 44.05, 27.80 ppm. Anal. calculated for C₁₃₇H₁₀₄N₈O₄: C, 85.42; H, 5.44; N, 5.82; found: C, 85.67; H, 5.49; N, 5.84. C₁₃₇H₁₀₄N₈O₄[M⁺] exact mass = 1924.82, MS (ESI) = 1925.76.

### Example 5

### Differential Scanning Calorimetry Measurements

For the evaluation of the ability to undergo a crosslinking process of the synthesized 9,9'-spirobisfluorene-based compounds and mixtures, including 9,9'-spirobifluorene compound and compound with two thiol groups (4,4'-thiobisbenzenethiol), thermal properties were studied by means of differential scanning calorimetry (DSC). DSC was performed on a TA Instruments Q2000 differential scanning calorimeter under nitrogen atmosphere. The heating and cooling rate was 10 °C min⁻¹.

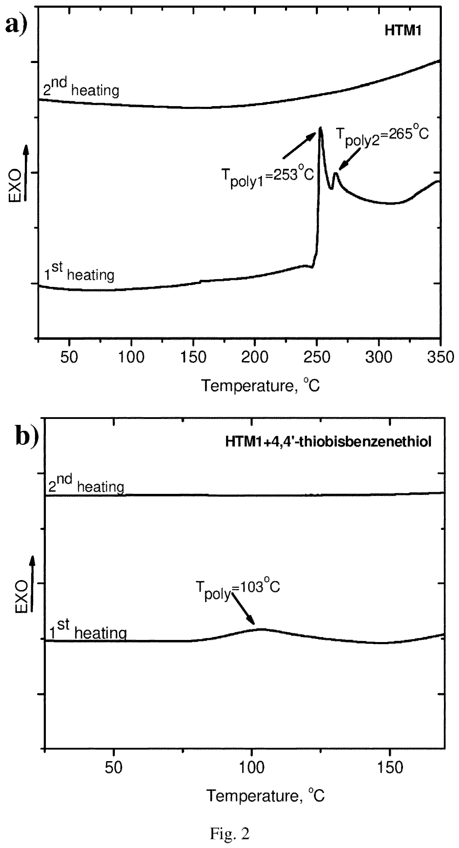

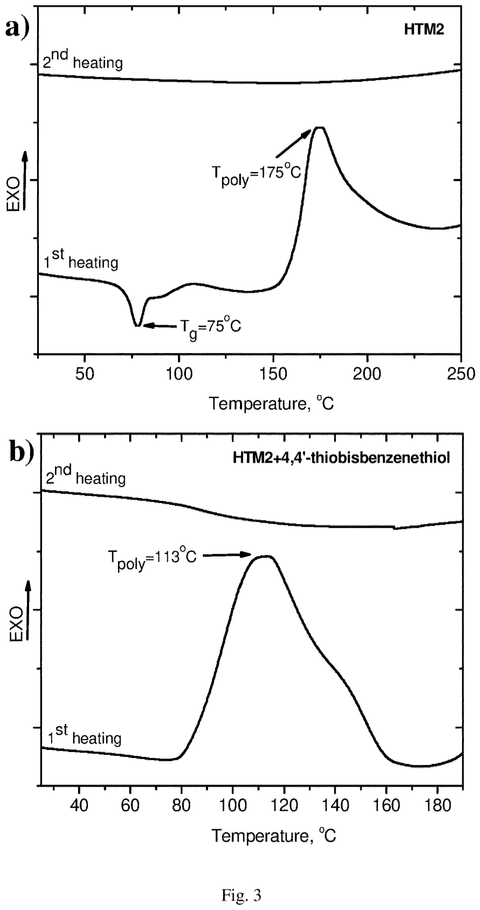

The DSC curves have shown that both **HTM1** and **HTM2** compounds exist only in an amorphous state since no endothermic melting peaks were detected during both heating cycles (Fig. 2a and Fig. 3a). For **HTM1,** during the first DSC heating cycle, only an exothermic process at 253 °C, suggesting that thermal polymerization occurred at this temperature. During the second heating cycle, no phase transitions were observed, confirming the formation of the crosslinked polymer (Fig. 2a). For compound **HTM2,** with higher molecular weight, the glass transition process was detected at 75 °C and the cross-linking process started at ~150 °C with a peak at ~175 °C (Fig. 3a). Again, during the second heating cycle, no phase transitions were detected.

Fig. 2b and Fig. 3b indicate that the thiol-ene click chemistry reaction between 9,9'-spirobifluorene compounds **HTM1** and **HTM2** decrees the crosslinking temperature drastically (103 °C for **HTM1** and 113 °C for **HTM2),** therefore the materials are suitable for the application in both n-i-p and p-in PSCs architecture.

### Example 6

### Estimation of Polymerization Conversion

The glass substrate was cleaned by sonication in TICKOPUR R 30 neutral cleaner (3 %) and subsequently rinsed in distilled water and acetone. Solutions of HTMs for spin-coating were prepared in tetrahydrofuran (20 mg/ml). Sample solutions (80 µl) were dropped onto pre-cleaned glass substrates (2.5 cm × 2.5 cm) and spin-coated at 3500 rpm for 30 s. The resulting films were heated on a hot plate in the air atmosphere at a temperature of crosslinking for different periods of time (0, 15, 30, 45, and 60 minutes). After cooling, films were separately dipped into tetrahydrofuran (5 ml) for 15 minutes. Resulting solutions were further used to record UV/vis spectra to evaluate the amount of the washed HTM to estimate polymerization conversion.

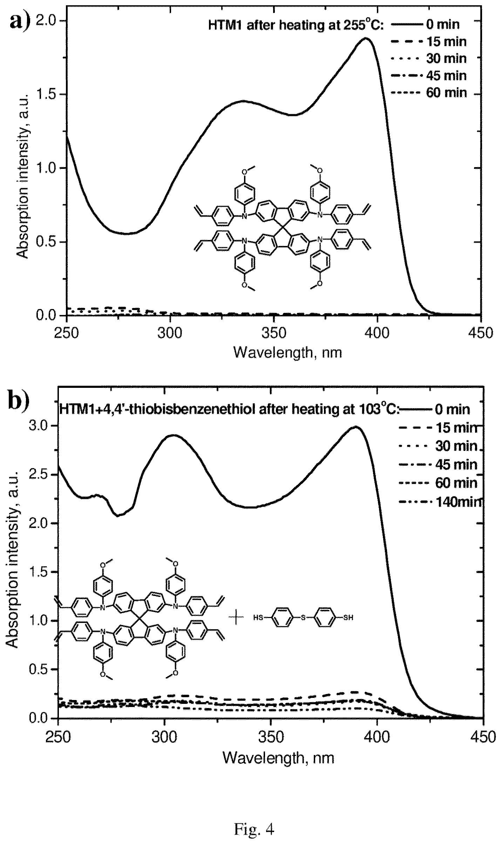

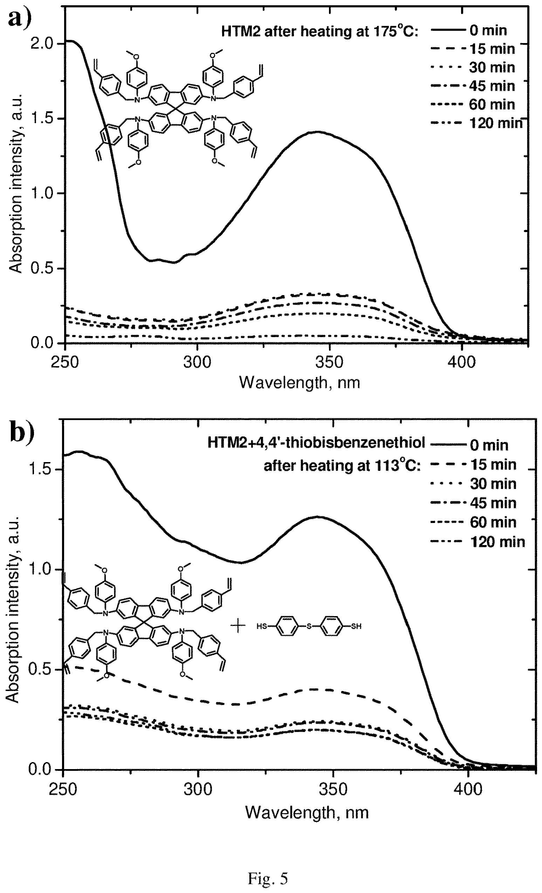

UV-vis results plotted in Fig. 4a,b and Fig. 5a,b suggest that the solvent resistance of crosslinked 9,9'-spirobisfluorene compounds and mixtures, including 9,9'-spirobifluorene compound and compound possessing two thiol groups, were 90-100 % after 15 minutes of the heating. This demonstrate that synthesized crosslinkable 9,9'-spirobisfluorene compounds can form an ideal crosslinked 3D network film.

### Example 7

### Ionization Potential Measurements

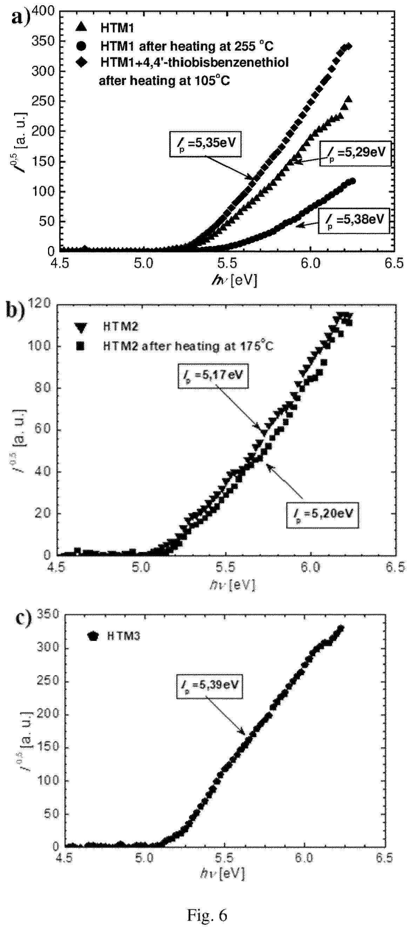

The solid-state ionization potential (*Iₚ*) of the layers of the synthesized 9,9'-spirobisfluorene-based compounds **HTM1-HTM4** and mixtures, including 9,9'-spirobifluorene compound and compound possessing two thiol groups (4,4'-thiobisbenzenethiol) was measured by the electron photoemission in air method (E. Miyamoto, Y. Yamaguchi, M. Masaaki, Electrophotography, 1989, vol. 28, pp. 364). The samples for the ionization potential measurement were prepared by dissolving materials and mixtures, including 9,9'-spirobifluorene compounds and compounds possessing two thiol groups in THF and were coated on Al plates pre-coated with -0.5 µm thick methylmethacrylate and methacrylic acid copolymer adhesive layer. The thickness of the transporting material layer was 0.5⁻¹ µm. In order to obtain the ionization potential of the crosslinked structures, the resulting layer is heated to a certain temperature for 30 min. Photoemission experiments are carried out in vacuum, and high vacuum is one of the main requirements for these measurements. If vacuum is not high enough the sample surface oxidation and gas adsorption influence the measurement results. In our case, however, the organic materials investigated are stable enough to oxygen, and the measurements may be carried out in the air. The samples were illuminated with monochromatic light from the quartz monochromator with deuterium lamp. The power of the incident light beam was (2-5)·10⁻⁸W. The negative voltage of -300 V was supplied to the sample substrate. The counter-electrode with the 4.5×15 mm² slit for illumination was placed at 8 mm distance from the sample surface. The counter-electrode was connected to the input of the BK2-16 type electrometer, working in the open input regime for the photocurrent measurement. A 10⁻¹⁵ - 10⁻¹² A strong photocurrent was flowing in the circuit under illumination. The photocurrent *I* is strongly dependent on the incident light photon energy *hυ.* The *I^{0.5}* =*f(hv)* dependence was plotted. Usually, the dependence of the photocurrent on incident light quanta energy is well described by linear relationship between *I*^{*0.*5} and hv near the threshold. The linear part of this dependence was extrapolated to the hv axis and *Iₚ* value was determined as the photon energy at the interception point. The *I*ₚ results are presented in Table 1 and Fig. 6.

### Example 8

### Hole Drift Mobility Measurements

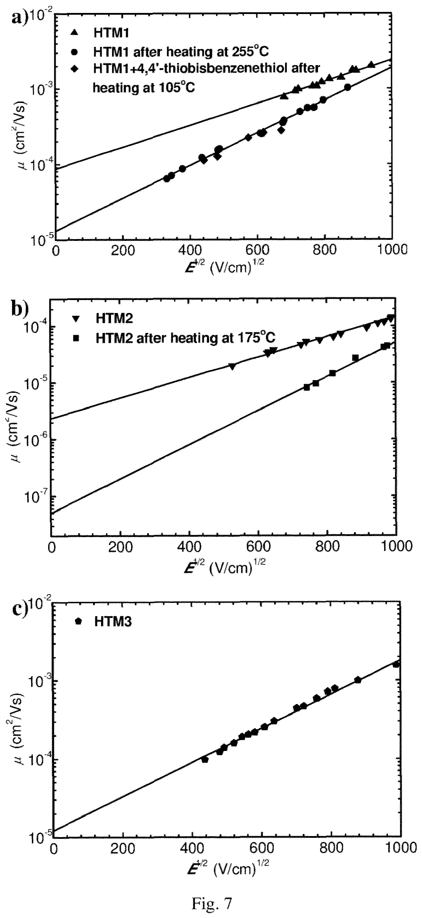

The samples for the hole mobility measurements were prepared by spin-coating the THF solutions of the synthesized 9,9'-spirobisfluorene-based compounds and mixtures, including 9,9'-spirobifluorene compound and compound possessing two thiol groups (4,4'-thiobisbenzenethiol) on the polyester films with conductive Al layer. The layer thickness was in the range of 2-5 µm. The hole drift mobility was measured by xerographic time of flight technique (XTOF) (Vaezi-Nejad, S. M., Int. J. Electronics, 1987, 62, No 3, 361-384). In order to obtain the hole mobility of the crosslinked structures, the resulting layer is heated to a certain temperature for 30 min. An electric field was created by positive corona charging. The charge carriers were generated at the layer surface by illumination with pulses of nitrogen laser (pulse duration was 2 ns, wavelength 337 nm). The layer surface potential decrease as a result of pulse illumination was up to 1-5% of initial potential before illumination. The capacitance probe that was connected to the wide frequency band electrometer measured the speed of the surface potential decrease d*U*/d*t.* The transit time *t*ₜ was determined by the kink on the curve of the d*U*/d*t* transient in double logarithmic scale. The drift mobility was calculated by the formula *µ*=*d*²/*U*₀*t*ₜ, where d is the layer thickness and *U*₀ is the surface potential at the moment of illumination. The *µ* results are presented in Table 1 and Fig. 7.

**Table 1. Ionization potential (Iₚ) and charge mobility values (µ) of the hole transporting compounds HTM1-HTM4, crosslinked HTM1-HTM4, and crosslinked mixtures, including 9,9'-spirobifluorene compound and 4,4'-thiobisbenzenethiol, 1:2.**

| **Compound or composition** | ***I*ₚ, eV** | **Mobility *µ*₀, cm²V⁻¹s⁻¹ (at 0 V/cm)** | **Mobility, cm²V⁻¹s⁻¹ (at 6.4·10⁵ V/cm)** |
|---|---|---|---|
| **HTM1** | 5.29 | 8.7×10⁻⁵ | 1.2×10⁻³ |
| **Crosslinked HTM1** | 5.38 | 1.3×10⁻⁵ | 7.2×10⁻⁴ |
| **Crosslinked HTM1+dithiol** | 5.35 | 1.3×10⁻⁵ | 7.2×10⁻⁴ |
| **HTM2** | 5.17 | 2.4×10⁻⁶ | 6.5×10⁻⁵ |
| **Crosslinked HTM2** | 5.20 | 5.0×10⁻⁸ | 1.3×10⁻⁵ |
| **HTM3** | 5.39 | 1.2×10⁻⁵ | 6.6×10⁻⁴ |
| **Spiro-OMeTAD** | 5.0 | 4.2×10⁻⁵ | 5.2×10⁻⁴ |

The estimated *I*ₚ values of synthesized 9,9'-spirobifluorene-based compounds are in the range of 5.17 eV to 5.39 eV and are slightly higher than the value of Spiro-OMeTAD (5.0 eV). The measured charge mobility values of synthesized compounds **HTM1-HTM3,** and crosslinked **HTM1-HTM3** are also comparable to the values measured for Spiro-OMeTAD, while charge mobility of the compound **HTM1** increases twofold in weak electric fields.

### Example 10

### Inverted p-i-n Perovskite Photovoltaic Cell Manufacture and Performance Measurements

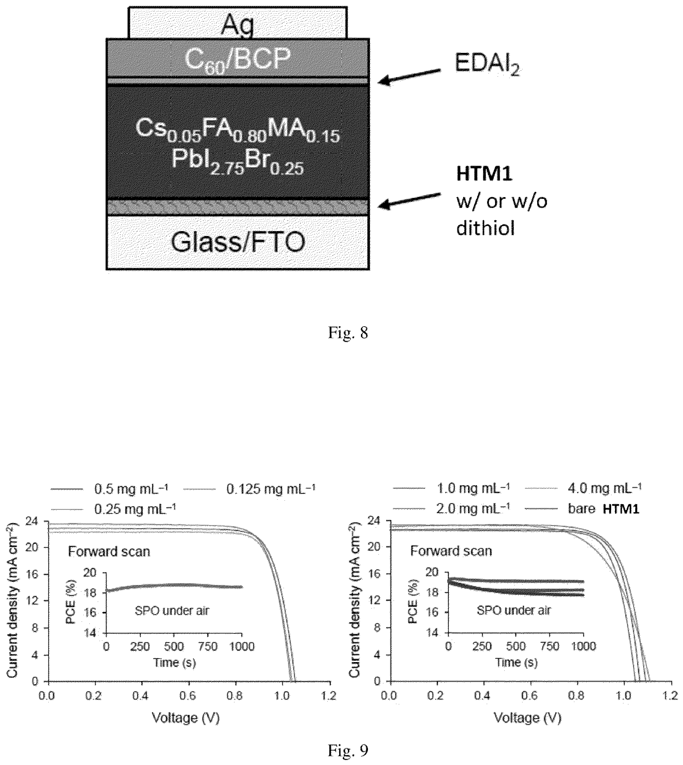

The performance of hole transporter compound **HTM1** was tested in mixed perovskite-based solar cells using a FTO photo-anode and an Ag cathode (FTO/**HTM1**/mixed perovskite/EDAI₂/C₆₀-BCP/Ag) (Fig. 8).

### Preparation of Transparent Conductive Oxide Substrates

Glass/FTO substrates (10 Ω sq⁻¹, AGC Inc.) were etched with zinc powder and HCl (6 M in deionized water), and consecutively cleaned with 15 min ultrasonic bath in water, acetone, detergent solution (Semico Clean 56, Furuuchi chemical), water, and isopropanol, followed by drying with an air gun, and finally plasma treatment. The substrates were transferred to an inert gas filled glove box for further processing.

### Preparation of Cross-Linkable Hole Transporting Layers

**HTM1** was mixed with 4,4'-thiobisbenzenethiol (molar ratio = 1:2, concentration of **HTM1** = 0.125-4 mg mL⁻¹) in chlorobenzene. The **HTM1** solution (100 µL) was deposited on FTO substrate using spin-coating (3000 rpm for 30 s, 5 s acceleration), followed by heating on a hot plate at 110 °C for 1 h. In case of bare **HTM1,** 8 mg mL⁻¹ **of HTM1** was used.

### Preparation of Perovskite Layer

The Cs_{0.05}FA_{0.80}MA_{0.15}PbI_{2.75}Br_{0.25} precursor solution was prepared from CsI (69 mg, 0.27 mmol), MABr (85 mg, 0.76 mmol), PbI₂ (2.24 g, 4.85 mmol), PbBr₂ (96 mg, 0.26 mmol), and FAI (703 mg, 4.09 mmol) dissolved in a mixture of DMF (3.0 mL) and DMSO (0.90 mL).

After stirring at 40 °C for 30 min, the solution was filtered with a 0.45 µm PTFE filter. 190 µL of the solution was placed on a FTO/HTM substrate and spread by spin-coating (slope 1 s, 1000 rpm 10 s, slope 5 s, 6000 rpm 20 s, slope 1 s) to make a thin film. 300 µL of chlorobenzene was dripped over the rotating substrate at 3 secs before the end of the spinning at 6000 rpm. The films were then annealed on a hot plate at 150 °C for 10 min.

The above perovskite samples were moved under Ar to a vacuum deposition chamber, where 0.5 nm of ethylenediammonium diiodide (EDAI₂) (deposition rate 0.03 nm s⁻¹) was deposited by thermal evaporation.

### Preparation of Electron Transporting Layer and Metal Electrode

The above samples were moved under Ar to a vacuum deposition chamber, where 20 nm of C₆₀ (deposition rate 0.05 nm s⁻¹) and 8 nm of BCP (deposition rate 0.01 nm s⁻¹) were deposited by thermal evaporation.

The top electrode was prepared by depositing 100 nm of silver (deposition rate 0.005 nm s⁻¹) through a shadow mask.

### Performance Measurements of Inverted p-i-n Perovskite Photovoltaic Cell

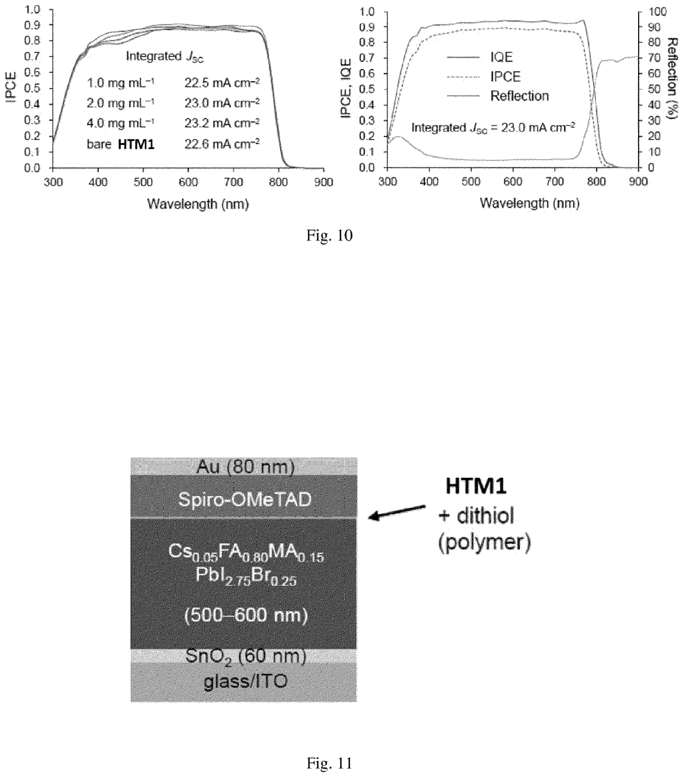

The fabricated perovskite photovoltaic cells were evaluated by photocurrent density-voltage (*J*-*V*) characteristics. The results, as shown in Fig. 9 and Fig. 10 show the performance of PV cells comprising different concentrations of **HTM1** tuning the thickness of the resulting layer. Table 2 shows summarized photovoltaic parameters. The highest PCE of 19.3% was obtained having 2 mg mL⁻¹ concentration of **HTM1** and using the dithiol crosslinker, while bare **HTM1** showed PCE of 18.8%.

### Example 11

### Conventional n-i-p Perovskite Solar Cell Module Manufacture and Performance Measurements

The performance of **HTM1** as an interlayer in a perovskite solar cell module was used in a module with a standard HTM, Spiro-OMeTAD. (ITO/SnO₂/mixed perovskite/**HTM1**/spiro-OMeTAD/Au), schematically shown in Fig. 11.

### Preparation of Transparent Conductive Oxide Substrates

Glass/ITO substrates (10 Ω sq⁻¹) were etched with zinc powder and HCl (6 M in de-ionized water), and consecutively cleaned with 15 min ultrasonic bath in water, acetone, detergent solution (Semico Clean 56, Furuuchi chemical), water, and isopropanol, followed by drying with an air gun, and finally plasma treatment. The substrates were transferred to an inert gas filled glove box for further processing.

**Table 2. Photovoltaic cell performance of compound HTM1**

| Concentration (mg mL⁻¹) | Scan | *J_{SC}* (mA·cm⁻²) | *V_{OC}* (V) | FF | PCE (%) |
|---|---|---|---|---|---|
| 0.125 | F | 22.2 (22.5 ±0.2) | 1.03 (0.99 ±0.03) | 0.81 (0.80 ±0.02) | 18.6 (17.8 ±0.7) |
| | R | 20.0 (20.2 ±0.3) | 1.01 (1.01 ±0.01) | 0.65 (0.66 ±0.02) | 13.2 (13.5 ±0.7) |
| 0.25 | F | 23.5 (22.5 ±0.7) | 1.04 (1.03 ±0.01) | 0.79 (0.79 ±0.01) | 19.2 (18.3 ±0.7) |
| | R | 22.8 (21.1 ±1.2) | 1.05 (1.02 ±0.02) | 0.75 (0.68 ±0.04) | 18.0 (14.6 ±1.9) |
| 0.5 | F | 22.9 (22.3 ±1.1) | 1.05 (1.03 ±0.01) | 0.80 (0.78 ±0.02) | 19.1 (18.0 ±0.9) |
| | R | 22.2 (21.7 ±1.1) | 1.05 (1.03 ±0.01) | 0.75 (0.70 ±0.04) | 17.5 (15.8 ±1.7) |
| 1 | F | 22.5 (21.8 ±0.6) | 1.06 (1.08 ±0.01) | 0.79 (0.78 ±0.01) | 19.0 (18.4 ±0.5) |
| | R | 22.5 (22.1 ±0.4) | 1.03 (1.06 ±0.02) | 0.72 (0.71 ±0.02) | 16.6 (16.5 ±0.7) |
| 2 | F | 23.0 (22.8 ±0.3) | 1.09 (1.08 ±0.01) | 0.77 (0.76 ±0.02) | 19.3 (18.7 ±0.5) |
| | R | 23.5 (23.0 ±0.3) | 1.07 (1.07 ±0.01) | 0.75 (0.75 ±0.02) | 18.8 (18.6 ±0.5) |
| 4 | F | 23.3 (23.1 ±0.3) | 1.11 (1.09 ±0.01) | 0.67 (0.65 ±0.02) | 17.3 (16.5 ±0.6) |
| | R | 23.3 (23.1 ±0.2) | 1.10 (1.09 ±0.01) | 0.71 (0.72 ±0.01) | 18.2 (18.0 ±0.2) |
| 8 (bare) | F | 22.5 (22.4 ±0.2) | 1.05 (1.06 ±0.01) | 0.79 (0.78 ±0.03) | 18.5 (18.4 ±0.4) |
| | R | 22.3 (22.2 ±0.1) | 1.06 (1.06 ±0.01) | 0.79 (0.75 ±0.05) | 18.8 (17.6±1.1) |

### Preparation of the SnO₂ Layer

The SnO₂ layer was prepared by spin-coating a colloidal dispersion (15% in H₂O) diluted with deionized water (volume ratio = 1:1) on the ITO substrates (400 µL for each substrate, slope 2 s, 3000 rpm 20 s, slope 2 s) followed by annealing at 150 °C for 30 min. A plasma treatment was performed after cooling the substrate to room temperature, before transferring the samples to an inert gas filled glove box for further processing.

### Preparation of Perovskite Layer

The Cs_{0.05}FA_{0.80}MA_{0.15}PbI_{2.75}Br_{0.25} precursor solution was prepared from CsI (69 mg, 0.27 mmol), MABr (85 mg, 0.76 mmol), PbI₂ (2.24 g, 4.85 mmol), PbBr₂ (96 mg, 0.26 mmol), and FAI (703 mg, 4.09 mmol) dissolved in a mixture of DMF (3.0 mL) and DMSO (0.90 mL).

After stirring at 40 °C for 30 min, the solution was filtered with a 0.45 µm PTFE filter. 190 µL of the solution was placed on a FTO/HTM substrate and spread by spin-coating (slope 1 s, 1000 rpm 10 s, slope 5 s, 6000 rpm 20 s, slope 1 s) to make a thin film. 300 µL of chlorobenzene was dripped over the rotating substrate at 3 s before the end of the spinning at 6000 rpm. The films were then annealed on a hot plate at 150 °C for 10 min.

### Preparation of HTM1 Interlayer

HTM1 was mixed with 4,4'-thiobisbenzenethiol (molar ratio = 1:2, concentration of HTM1 = 1.0, 2.0 mg mL⁻¹) in chlorobenzene. 100 µL of the solution was spin-coated on top of the perovskite layer (3000 rpm for 30 s, 5 s acceleration), followed by heating on a hot plate at 110 °C for 1 h.

### Preparation of Hole-Transporting Layer

Spiro-OMeTAD (0.06 M) was mixed with an oxidizing agent [tris(2-(1*H*-pyrazol-1-yl)-4-*tert-*butylpyridine)cobalt(III) tris(bis(trifluoromethylsulfonyl)imide)] (FK209, 0.15 equiv) into a solution of chlorobenzene, 4-tert-butylpyridine (tBP, 3.3equivalents), and lithium bis(trifluoromethylsulfonyl)imide (LITFSI, 0.54 equiv). After stirring at 70 °C for 30 min, the suspension was filtered with a 0.45 µm PTFE filter to remove insoluble Co(II) complexes. 90 µL of the solution was spin-coated on top of V1382 (slope 4 s, 4000 rpm, 30 s, slope 4 s), followed by annealing at 70 °C for 30 min.

### Preparation of Metal Electrode

Gold electrodes (80 nm) were thermally deposited on the top face of the devices using a shadow mask.

### Performance Measurements of Conventional n-i-p Perovskite Photovoltaic Cell

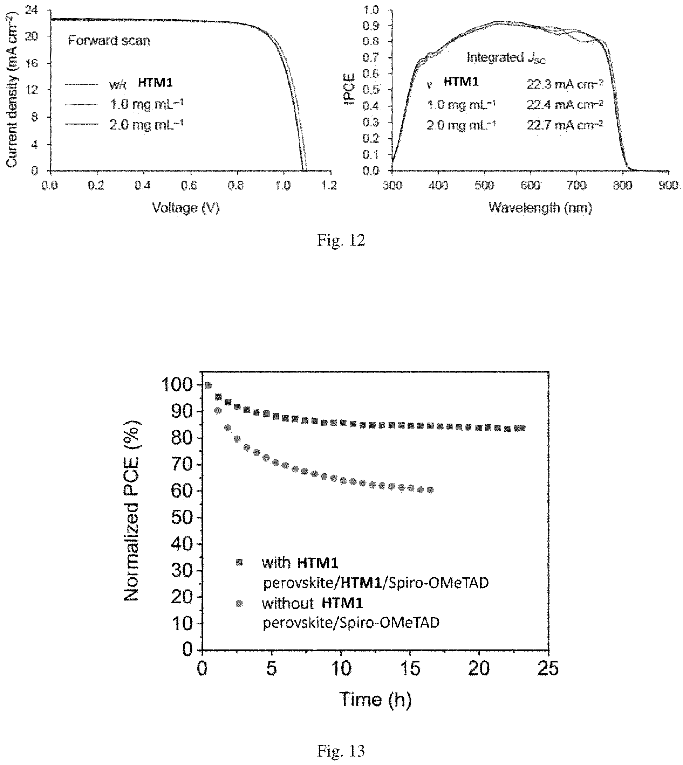

The fabricated perovskite photovoltaic cells were evaluated by photocurrent density-voltage (J-V) characteristics. The results, as shown in Fig. 12 show the performance of PV cells comprising **HTM1** acting as interlayer. Table 3 shows photovoltaic parameters extracted from J-V curves. Having the **HTM1** interlayer in the device structure improved the PCE to 19.1 %, while device without **HTM1** showed PCE of 18.9%. Fig. 13 shows the maximum power point tracking (MPPT), which indicates that having the **HTM1** interlayer improves the stability of the device performance.

**Table 3. Performance of Photovoltaic cell containing compound HTM 1.**

| Concentration (mg mL⁻¹) | Scan | *J_{SC}* (mA·cm⁻²) | *V_{OC}* (V) | FF | PCE (%) |
|---|---|---|---|---|---|
| w/o HTM1 | F | 22.6 (22.2 ±0.4) | 1.08 (1.05 ±0.02) | 0.77 (0.75 ±0.01) | 18.9 (17.5 ±0.8) |
| | R | 22.5 (22.1 ±0.4) | 1.07 (1.06 ±0.01) | 0.75 (0.74 ±0.01) | 18.0 (17.4 ±0.5) |
| 1 | F | 22.4 (22.1 ±0.4) | 1.10 (1.05 ±0.03) | 0.77 (0.75 ±0.01) | 19.1 (17.6 ±0.8) |
| | R | 22.4 (22.1 ±0.4) | 1.09 (1.06 ±0.02) | 0.76 (0.76 ±0.01) | 18.5 (17.8 ±0.5) |
| 2 | F | 22.7 (22.3 ±0.3) | 1.08 (1.07 ±0.01) | 0.77 (0.76 ±0.01) | 18.8 (18.2 ±0.4) |
| | R | 22.6 (22.2 ±0.3) | 1.07 (1.07 ±0.01) | 0.75 (0.76 ±0.01) | 18.3 (18.0 ±0.2) |

The following patents, applications, and publications as listed below and throughout this document are hereby incorporated by reference in their entirety herein.

### REFERENCES

[1] Best Research-cell Efficiencies Chart, https://www.nrel.gov/pv/ assets/pdfs/best-research-cell-efficiencies.20200925.pdf
[2] J. Ramanujam, U. P. Singh, Energy Environ. Sci. 2017, 10, 1306.
[3] Y. M. Yang, A. Yu, B. Hsu, W. C. Hsu, A. Yang, C. W. Lan, Prog. Photovoltaics 2015, 23, 340.
[4] L. Chao, T. Niu, W. Gao, C. Ran, L. Song, Y. Chen, W. Huang, Adv. Mater. 2021, 33, 2005410.
[5] F. Fu, J. Li, T. C. Yang, H. Liang, A. Faes, Q. Jeangros, C. Ballif, Y. Hou, Adv. Mater. 2022, 34, 2106540.
[6] J.Y. Kim, J.W. Lee, H.S. Jung, H. Shin, N.G. Park, Chem Rev, 2020, 120:7867-7918.
[7] H. Min, M. Kim, S.-U. Lee, H. Kim, G. Kim, K. Choi, J. H. Lee, S. I. Seok, Science 2019, 366, 749.
[8] Y. Li, L. Meng, Y. M. Yang, G. Xu, Z. Hong, Q. Chen, J. You, G. Li, Y. Yang, Y. Li, Nat. Commun. 2016, 7, 10214.
[9] T. H. Schloemer, J. A. Christians, J. M. Luther, A. Sellinger, Chem. Sci. 2019, 10, 1904.
[10] X. Yang, J. Xi, Y. Sun, Y. Zhang, G. Zhou, W.-Y. Wong, Nano Energy 2019, 64, 103946.
[11] H. Park, R. Chaurasiya, B. H. Jeong, P. Sakthivel, H. J. Park, Adv. Photonics Res. 2021, 2, 2000178.
[12] G. Ren, W. Han, Y. Deng, W. Wu, Z. Li, J. Guo, H. Bao, C. Liu, W. Guo. J. Mater. Chem. A, 2021, 9, 4589.
[13] Z. Hu, W. Fu, L. Yan, J. Miao, H. Yu, Y. He, O. Goto, H. Meng, H. Chen, W. Huang. Chem. Sci., 2016, 7, 5007.
[14] M. Stolterfoht, C. M. Wolff, J. A. Márquez, S. Zhang, C. J. Hages, D. Rothhardt, S. Albrecht, P. L. Burn, P. Meredith, T. Unold and D. Neher. Nat. Energy, 2018, 3, 847.
[15] W. Li, H. Wang, X. Hu, W. Cai, C. Zhang, M. Wang and Z. Zang, Sol. RRL, 2021, 5, 2000573.
[16] X. Sun, Z. Zhu, Z. Li. Frontiers of Optoelectronics, (2022) 15:46.

## Claims

1. A compound of formula **(I)** 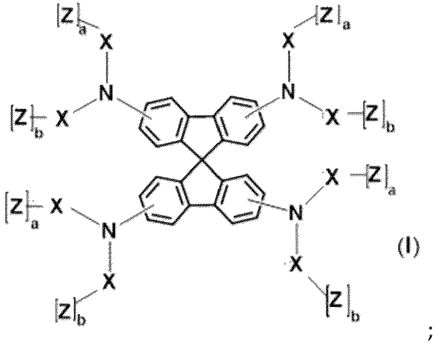 wherein
- X is independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said alkyl, alkenyl, alkynyl moieties, if they comprise 3 or more carbons, may be linear, branched or cyclic, and said alkyl, alkenyl, alkynyl, aryl, alkylaryl, alkenylaryl, alkynylaryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl, C4 to C10 heteroaryl, or one or more heteroatoms being selected from N, S and O;
- *a* and *b* are an integer independently being 0 or 1, wherein *a*+*b* ≥ 1; and
- Z is selected from C2-C10 alkenyl, C2-C10 alkynyl, C4-C20 alkenylaryl, acetylenyl group, alkenyloxy alkyl group, -SH, acrylate group, -OH, -COOH, urethane group, ethyl ester group, C4-C20 alkoxyalkenyl, azide group, epoxy compounds, methyl oxirane group, epoxy group, oxiranyl group, and oxetanyl group, wherein Z is independently selected for groups [Z]ₐ and [Z]_{b}.

2. The compound of formula **(I)** according to claim 1, wherein *a* is 1 and X is selected from C1-C10 alkyl and C4-C10 aryl.

3. The compound of formula **(I)** according to claim 1, wherein Z is selected from a moiety according to anyone of formulae (1)-(4): 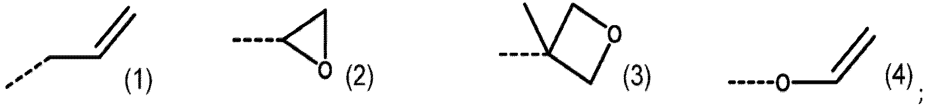 wherein the dotted line represents a single bond between the substituent X of compound of formula **(I)** or between 9,9'-spirobifluorene core of the compound of formula **(I),** when *a* is 0, and anyone of the moieties is (1)-(4).

4. An optoelectronic and/or photoelectrochemical device comprising a hole transporting material comprising a polymer film of formula (Ia): 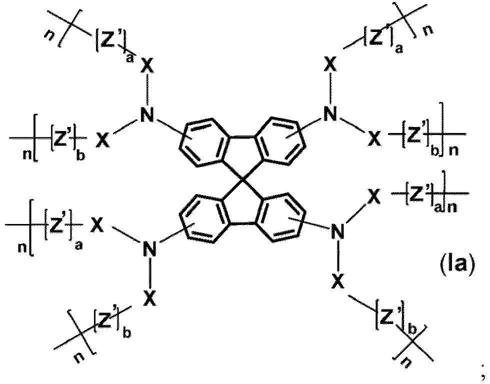 wherein
- X is independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said alkyl, alkenyl, alkynyl moieties, if they comprise 3 or more carbons, may be linear, branched or cyclic, and said alkyl, alkenyl, alkynyl, aryl, alkylaryl, alkenylaryl, alkynylaryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl, C4 to C10 heteroaryl, or one or more heteroatoms being selected from N, S and O;
- *a* and *b* are an integer independently being 0 or 1, wherein *a*+*b* ≥ 1;
- Z' is selected from a moiety according to anyone of formulae (1')-(4'): 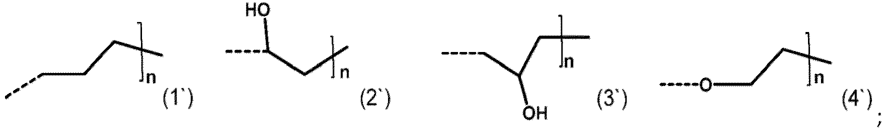 and
- n is 2 or more.

5. The optoelectronic and/or photoelectrochemical device of claim 4, wherein the Z' moiety is crosslinked with one of (6)-(16):

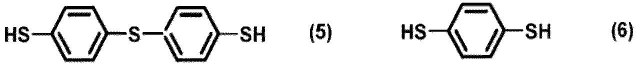

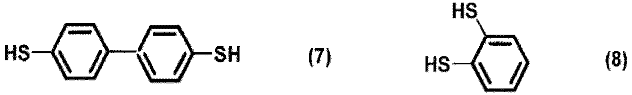

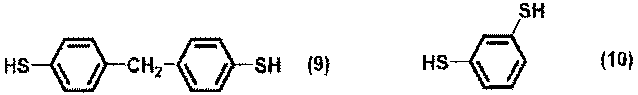

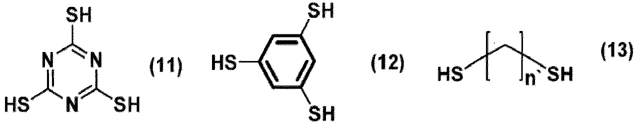

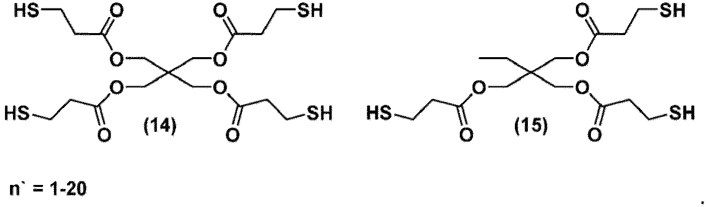

6. The optoelectronic and/or photoelectrochemical device of claim 4, wherein a first and second monomer of formula (Ia) of the polymer film are identical or a mixture of monomers with the proviso that Z' is identical between the all monomers.

7. The optoelectronic and/or photoelectrochemical device of claim 4, wherein the hole transporting material is provisioned as a hole transport layer in the optoelectronic and/or photoelectrochemical device.

8. The optoelectronic and/or photoelectrochemical device of claim 4, wherein the hole transport layer has a thickness in the range from 20 to 400 nm.

9. The optoelectronic and/or photoelectrochemical device of claim 4, comprising a layer underlying the hole transport layer being selected from a hole injection layer, a sensitizer layer, a light-harvester layer, or a conducting current collector, wherein said underlying layer is not crosslinked.

10. The optoelectronic and/or photoelectrochemical device of claim 4, wherein the device is selected from a photovoltaic device, an organic photovoltaic device, a photovoltaic solid state device, an organic solar cell, a solid state solar cell, a perovskite solar cell, a light emitting electrochemical cells, and OLED.

11. The optoelectronic and/or photoelectrochemical device of claim 10 is selected from a p-i-n perovskite and a n-i-p perovskite solar cell.

12. The optoelectronic and/or photoelectrochemical device of claim 10, wherein the polymer film of formula (Ia) is one of:
N²,N^{2'},N⁷,N^{7'}-tetrakis(4-methoxyphenyl)-N²,N^{2'},N⁷,N^{7'}-tetrakis(4-vinylphenyl)-9,9'-spirobi[fluorene]- 2,2',7,7'-tetraamine,
N²,N^{2'},N⁷,N^{7'}-tetrakis(4-methoxyphenyl)-N²,N^{2'},N⁷,N^{7'}-tetrakis(4-vinylbenzyl)-9,9'-spirobi[fluorene -2,2',7,7'-tetraamine,
N²,N^{2'},N⁷,N^{7'}-tetrakis(4-methoxyphenyl)-N²,N^{2'},N⁷,N^{7'}-tetrakis[4-(vinyloxy)phenyl]-9,9'-spirobi[fluorene]-2,2',7,7'-tetraamine, and
N²,N^{2'},N⁷,N^{7'}-tetrakis(4-methoxyphenyl)-N²,N^{2'},N⁷,N^{7'}-tetrakis[9-(4-vinylbenzyl)-9H-carbazol-3-yl]-9,9'-spirobi[fluorene]-2,2',7,7'-tetraamine.

13. A method for fabricating an optoelectronic and/or photoelectrochemical device comprising a hole transport layer as defined in claim 1, said method comprising:
- providing a layer before providing a hole layer, said layer being an underlying layer,
- providing the hole transport layer onto the underlying layer comprising,
- applying a hole transporting material comprising a polymer precursor by a liquid deposition process onto the underlying layer, and
- polymerizing the hole transporting material by thermal, chemical or irradiative means, and
- providing an overlaying layer;
wherein the polymer precursor is a compound of formula (I): 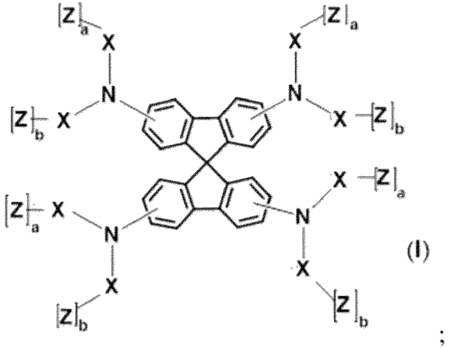 wherein
- X is independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said alkyl, alkenyl, alkynyl moieties, if they comprise 3 or more carbons, may be linear, branched or cyclic, and said alkyl, alkenyl, alkynyl, aryl, alkylaryl, alkenylaryl, alkynylaryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl, C4 to C10 heteroaryl, or one or more heteroatoms being selected from N, S and O;
- *a* and *b* are an integer independently being 0 or 1, wherein *a*+*b* ≥ 1;
- Z is selected from C2-C10 alkenyl, C2-C10 alkynyl, C4-C20 alkenylaryl, acetylenyl group, alkenyloxy alkyl group, -SH, acrylate group, -OH, -COOH, urethane group, ethyl ester group, C4-C20 alkoxyalkenyl, azide group, epoxy compounds, methyl oxirane group, epoxy group, oxiranyl group, and oxetanyl group, wherein Z is independently selected for groups [Z]ₐ and [Z]_{b}.

14. The method for fabricating an optoelectronic and/or photoelectrochemical device of claim 13, wherein a first Z moiety of a first precursor is bound to a second Z moiety of a second precursor.

15. The method for fabricating an optoelectronic and/or photoelectrochemical device of claim 13, wherein the first and second precursors of the polymer are identical or a mixture of different precursor compounds with the proviso that Z is identical between the first and second precursors.

16. The method for fabricating an optoelectronic and/or photoelectrochemical device of claim 13 further comprising providing a crosslinking agent with two or more thiol groups together with the polymer precursor.

17. The method for fabricating an optoelectronic and/or photoelectrochemical device of claim 16, wherein the crosslinking agent is chosen from compounds (6)-(16):

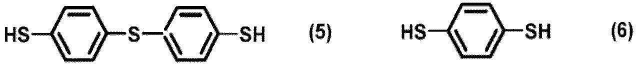

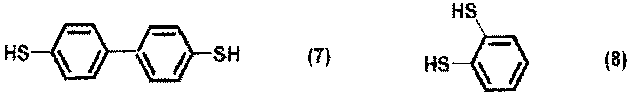

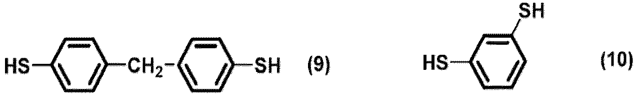

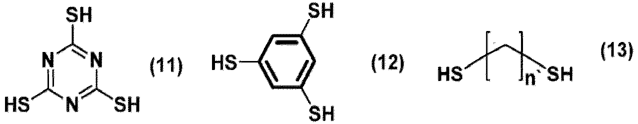

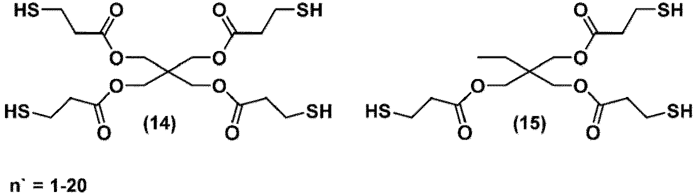

18. The method for fabricating an optoelectronic and/or photoelectrochemical device of claim 16, wherein the hole transport layer is polymerized by thermal means, and wherein the temperature does not exceed 110°C.

19. The method for fabricating an optoelectronic and/or photoelectrochemical device of claim 13, wherein the first layer underlying the hole transport layer is selected from a hole injection layer, a sensitizer layer, a light-harvester layer, or a conducting current collector, wherein said underlying layer is not crosslinked.

20. The method for fabricating an optoelectronic and/or photoelectrochemical device of claim 19, wherein said optoelectronic and/or photoelectrochemical device is an organic light-emitting diode, the underlying layer is a hole injection layer and the overlaying layer is an emissive layer.

21. The method for fabricating an optoelectronic and/or photoelectrochemical device of claim 19, wherein said optoelectronic and/or photoelectrochemical device is a solid state solar cell, wherein the underlying layer is a sensitizer layer or light-harvesting layer and the overlying layer is a counter electrode or a conducting current providing layer, or the device is a solid state solar cell, wherein the underlying layer is a conducting current collector and the overlying layer is a sensitizer layer or a light-harvesting layer.

22. The method for fabricating an optoelectronic and/or photoelectrochemical device of claim 19, wherein the sensitizer layer, the light-harvesting layer, or the emissive layer comprises an organic-inorganic perovskite.
